# EUROPEAN PATENT APPLICATION

(11) **EP 4 183 392 A1**
(43) Date of publication of application: **24.05.2023**
(21) Application number: 21752070.9
(22) Date of filing: 16.07.2021
(51) Int. Cl.: A61K 31/202, A61P 11/00, A61P 31/00, A61P 31/04, A61P 31/12, A61P 31/14, A61P 31/16, A23L 33/12, G01N 33/00

(54) **DIAGNOSTIC METHOD AND COMPOUNDS FOR THE TREATMENT OF INFECTIONS CAUSED BY A PATHOGEN**

(30) Priority: 17.07.2020 ES 202030752
(71) Applicant: Solutex GC, S. L., 50550 Mallén (Zaragoza) (ES)
(72) Inventor: MORENO EGEA, Fernando, 50550 Mallén (Zaragoza) (ES)
(74) Representative: Pons
(86) International application number: PCT/ES2021/070528
(87) International publication number: WO 2022/013478

(57) **Abstract**

The present invention provides a new family of compounds characterized as being long chain polyunsaturated fatty acids oxidized at certain positions, and which are useful for the treatment of infections caused by a pathogen in the respiratory system. Furthermore, the present invention shows a method of diagnosing and/or monitoring of this type of diseases to be treated with the compounds of the invention.

## Description

The present invention provides compounds and compositions that can be administered for the treatment of infections caused by a pathogen, infectious agent, or a pathogenic biological agent.

### STATE OF THE ART

Pneumonia or pneumonia is a disease of the respiratory system that consists of inflammation of the alveolar spaces of the lungs. Pneumonia can be classified based on the part of the body affected, for example, a complete lung lobe (lobar pneumonia), a segment of a lobe, the alveoli near the bronchi (bronchopneumonia) or the interstitial tissue (interstitial pneumonia). Pneumonia causes the lung tissue to look red, swollen, inflamed, and become painful. Pneumonia can be a serious disease if not caught early, and it can be fatal, especially among the elderly and the immunosuppressed. In particular, AIDS patients frequently contract *Pneumocystis.* People with cystic fibrosis are at high risk for pneumonia because fluid continually builds up in their lungs.

Pneumonia can also be classified based on who causes it, such as bacterial pneumonia, atypical pneumonia, and viral pneumonia.

In viral pneumonia, viruses reach the lung through inhaled drops or microdroplets or by ingestion through the nose and mouth, invading the cells that line the airways and alveoli. This invasion often leads to cell death, either directly or through apoptosis. When the immune system responds to the viral infection, lung damage worsens. Leukocytes, mainly lymphocytes, activate a variety of chemical mediators of inflammation, such as cytokines, which increase the permeability of the bronchial alveolar wall allowing the passage of fluids. The combination of cell destruction and the passage of fluids into the alveolus worsens gas exchange. In addition to lung damage, many viruses infect other organs and can interfere with multiple functions and causing other diseases. Viral infection can also make the host more susceptible to bacterial infection.

Among the causes of viral pneumonia are: influenza A and B virus, respiratory syncytial virus (RSV), human parainfluenza virus, adenovirus, metapneumovirus, severe acute respiratory syndrome coronavirus (SARS-CoV or SARS-CoV1), coronavirus severe acute respiratory syndrome 2 (SARS-CoV-2), Middle East respiratory syndrome coronavirus (MERS-CoV), coronavirus 229E (HCoV-229E), coronavirus NL63 (HCoV-NL63), coronavirus OC43 (HCoV-OC43), HKU1 coronavirus (HCoV-HKU1), and hantavirus. On the other hand, there are viruses that mainly cause other diseases, but that sometimes cause pneumonia, and where they include: herpes simplex viruses (HSV), varicella-zoster virus (VZV), measles virus, virus rubella, cytomegalovirus (CMV), mainly in people with immune system problems, smallpox virus, dengue virus. Viral pneumonia affects about 200 million people a year in the world.

Severe Acute Respiratory Syndrome (SARS) is a viral respiratory disease caused by a coronavirus, called SARS-associated coronavirus, for example, SARS-CoV1 and SARS-CoV2. SARS was first reported in Asia in 2003. Within months, SARS spread across three continents before the global outbreak could be contained. SARS generally begins with a high fever, above 38.0 °C. Other symptoms can be a headache, a general feeling of discomfort, and pain in the body. Some people experience mild respiratory symptoms early in the illness. After 2 to 7 days, SARS patients may develop a dry cough, and most develop pneumonia.

SARS-CoV-2 coronavirus is the cause of the current coronavirus infectious disease pandemic of 2019 or COVID-19 and shows many similarities with SARS (fever, cough, fatigue, shortness of breath and loss of smell), evolving in severe cases to viral pneumonia, cytokine storm and multi-organ failure. The latter has been associated, among others, with disseminated intravascular coagulation, or defibrination syndrome, which is a pathological process that occurs as a result of excessive thrombin formation, and induces the consumption of coagulation factors and platelets in the blood, which produces the appearance of hemorrhages in different parts of the body, obstructive thrombosis (microclots) of the microcirculation, necrosis and organic dysfunctions (Tang N., et al., "Anticoagulant treatment is associated with decreased mortality in severe coronavirus disease 2019 patients with coagulopathy", J. Thromb. Haemost., 2020; 18: 1094-9). The most worrisome symptoms include shortness of breath, persistent chest pain, confusion, trouble waking up, and bluish skin.

Currently, there is no specific treatment or vaccine for COVID-19.

As mentioned above, cytokine storm is a dangerous syndrome that can be triggered by overstimulation of cytokines during pneumonia, or in the particular case of COVID-19. Thus, a patient who apparently evolves favorably to COVID-19 can suddenly worsen and generate a picture of severe respiratory failure due to a sudden and hypertrophied reaction of the immune system that generates a large-scale lung inflammation. During a cytokine storm, the immune system responds disproportionately by producing massive inflammation in the respiratory system that, in some cases, ends up leading to respiratory failure and multi-organ failure.

Cytokines are proteins that regulate the function of the cells that produce them on other cell types. They are the agents responsible for a part of intercellular communication, they induce the activation of specific membrane receptors, functions of cell proliferation and differentiation, chemotaxis, growth and modulation of immunoglobulin secretion. They are produced mainly by activated lymphocytes and macrophages, although they can also be produced by polymorphonuclear leukocytes (PMN), endothelial cells, epithelial cells, adipocytes, muscle tissue (myocytes) and connective tissue. Cytokines secreted by lymphocytes are called lymphokines, those produced by macrophages (Mf) are monokines, etc. (depending on the type of cell). Its fundamental action consists of regulating the mechanism of inflammation. There are pro-inflammatory and anti-inflammatory cytokines.

Each cytokine binds to a specific cell surface receptor generating cell signaling cascades that alter cell function. This includes the up-regulation or down-regulation of various genes and their transcription factors that result in the production of other cytokines, an increase in the number of surface receptors for other molecules, or the suppression of their own effect through retro-regulation. The main cytokines that act in the nonspecific response or inflammation are: Interleukin 1 (IL-1), Tumor Necrosis Factor Alpha (TNF-α), Interleukin 8 (IL-8), Interleukin 12 (IL-12), Interleukin 16 (IL-16) and Interferons.

All of them are pro-inflammatory. IL-6 and IL-12 also act on specific immunity: IL-6 is an autocrine factor of B7 lymphocytes while IL-12 stimulates cytotoxic cellular immunity.

Specialized Proresolving Mediators (SPMs) are lipid mediators that act in the inflammation resolution process and, therefore, can modulate all those processes that occur with inflammation and that must be brought to homeostasis, avoiding thus its chronification. SPMs are produced by cells of the innate immune system through the enzymatic conversion of essential fatty acids such as arachidonic acid (AA), eicosapentaenoic acid (EPA), n-3 docosapentaenoic acid (DPA n-3) and docosahexaenoic acid (DHA).

SPMs can be classified into four families: lipoxins, resolvins, protectins, and maresins. These endogenous lipid mediators share basic physiological properties in regulating host responses to actively improve the resolution of inflammatory response mechanisms, such as: reduction of pro-inflammatory mediator production, limitation of neutrophil trafficking, stimulation of phagocytosis of apoptotic cells of macrophages, phagocytosis of bacteria and elimination of cellular debris, through the modulation of G-protein-coupled receptors (GPCR).

On the other hand, it has been shown that certain SPMs have a potent activity against lung infections and inflammation (Nat. Rev. Immunol. 2016, 16 (1): 51-67). Furthermore, SPMs induce an anti-inflammatory response by inhibiting granulocyte migration, disrupting the activation of sensory neurons, and dampening cytokine production by a variety of structural cells, including epithelial cells, endothelial cells, and fibroblasts. Specifically, Protectin D1 (PD1) limits the pathogenicity of influenza by interacting directly with the RNA replication machinery to inhibit nuclear export of viral RNA. In particularly virulent influenza strains, PD1 formation is insufficiently regulated, leading to more efficient viral replication and disappearance.

Although it has been observed that certain SPMs have antiviral activity, at no time has been described the antiviral activity of the maresin family described in the present invention.

Regarding maresin family, it has been proven that these compounds promote the resolution of acute inflammation (Serhan, C.N., et al., PCT/US2009/056998). Furthermore, a specific member, Maresin 1, has been shown to resolve inflammation in lung caused by an allergy (Krishnamoorthy, N. et al., J. Immunol. 2015, 194, 863-867), and reduce the proinflammatory response in the epithelial cells of the bronchi in the presence of organic dust (Nordgren, T.M., et al., Respir. Res. 2013, 14, 51).

For all of the above, there is a need to find drugs capable of modulating an adequate response of the body against external aggression by a pathogen. If this type of drug can also mitigate, alleviate and even prevent the appearance of the so-called cytokine storm, then it will be more effective in treating a patient affected by a disease, condition or syndrome caused by a pathogen.

In WO2018098244, Serhan C.N., et al., describe a diagnostic method based on the identification of clusters of lipid mediators such as, for example, certain prostaglandins and/or certain SPMs, and their quantification in blood and/or in the coagulation process of the blood. Thus, a certain cluster of lipid mediators, together with the measurement of their concentration levels, could be associated with a certain disease. An association of such characteristics may allow obtaining a diagnostic method and/or a method for monitoring the evolution of a given disease in a patient suffering from such disease. However, the determination of one or more clusters of lipid mediators that are characteristic of a certain disease is not obvious since to date dozens of lipid mediators (prostaglandins, thromboxanes, leukotrienes, SPMs) have been identified and are disseminated by the whole organism.

Obtaining a method of diagnosis and monitoring of the evolution of a patient affected by a disease caused by a pathogen, which occurs with lung inflammation and/or cytokine storm, such as pneumonia, viral pneumonia, or Covid-19, will provide a very useful tool for the diagnosis, treatment and monitoring of a patient suffering from a disease of these characteristics.

### DESCRIPTION OF THE INVENTION

The inventors of the present invention have surprisingly found a family of compounds that are unsaturated analogs of docosanoic acid that reduce the production of cytokines in lung tissue. Therefore, these compounds are useful for the treatment of infections caused by the presence of a pathogen in the respiratory system, and which are represented by the formulas: (I), (Ia), (Ib), (Ic), (Id), (le), (II), (IIa), (IIb), (IIc), (III), (IIIa), (IIIb), (IIIc), (IIId), (IV), (IVa), (IVb), (V), (Va), (Vb), (Vc), (Vd), (Ve), (Vf), (Vg), (VI), (VIa), (Vlb), ( VIc), (VId), and (VII); 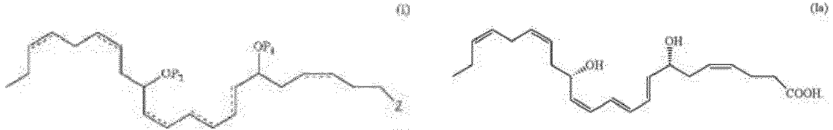 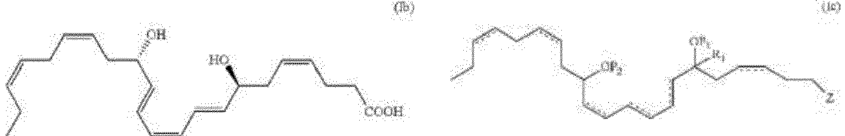 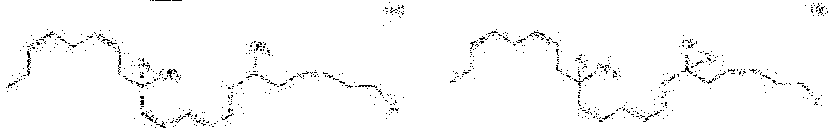 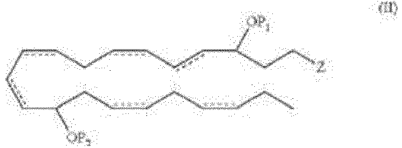 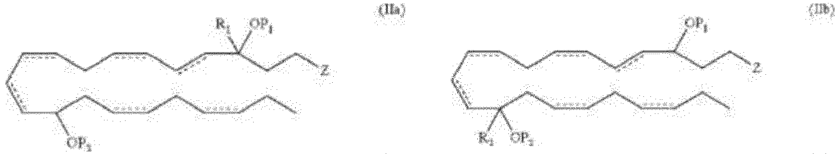 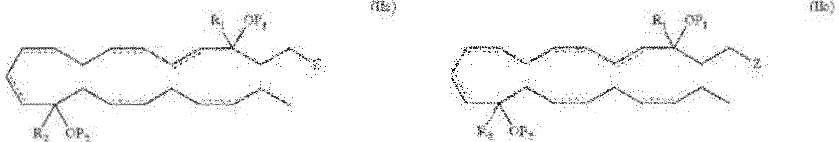 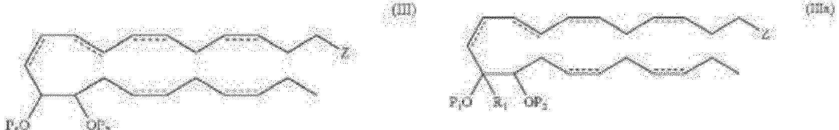 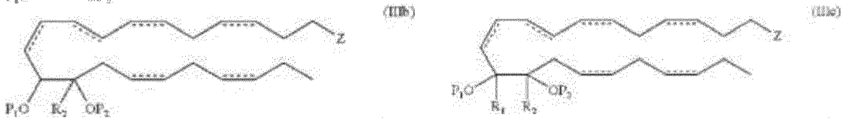 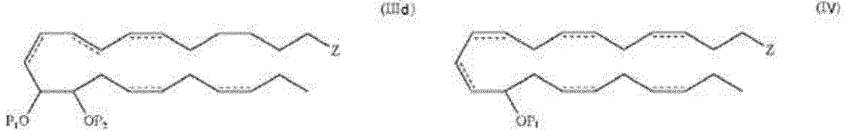 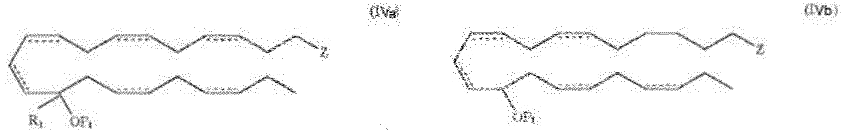 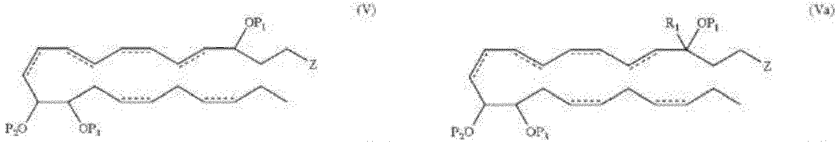 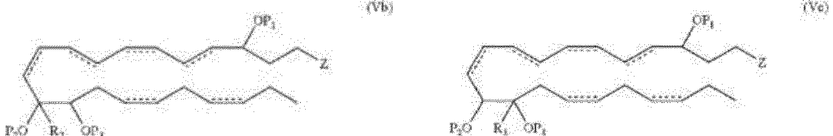 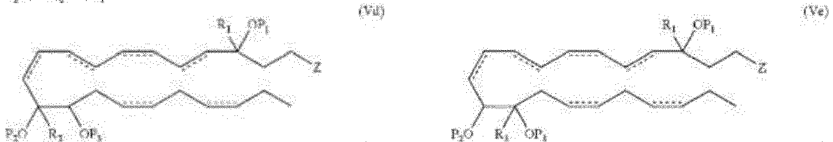 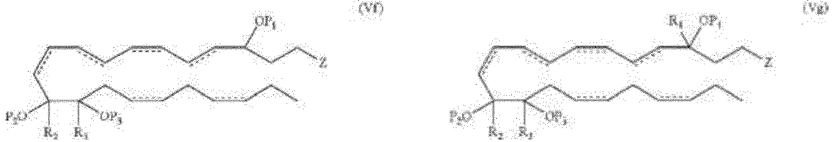 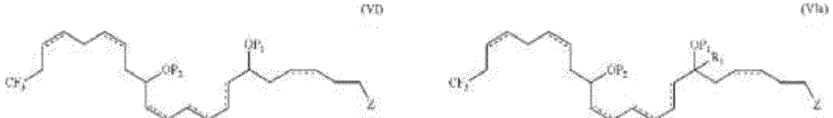 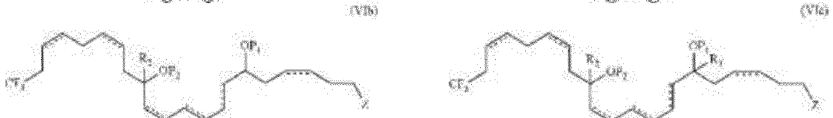 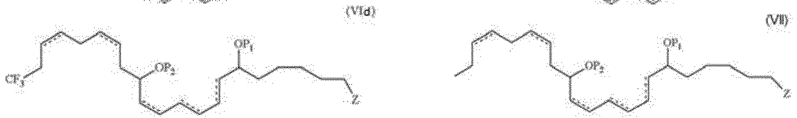
their pharmaceutically acceptable salts, tautomers, and/or solvates thereof;
where each of P₁ and P₂, individually, is a protecting group or a hydrogen atom;
wherein is -̅-̅-̅-̅-̅-̅ a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl;
each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy,-OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d},-[NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c}; each n, independently is an integer from 0 to 3;
each R^{d}, independently is a protecting group or R^{a};
wherein R₁ and R₂ are independently selected from (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl;
for use in the treatment of infections caused by the presence of a pathogen in the respiratory system.

In a second inventive aspect, the present invention comprises a method for diagnosing and/or monitoring the condition of a patient, suffering from an infection caused by the presence of a pathogen in the respiratory system, based on the identification of a cluster of pro-resolutive lipid mediators in the blood of said patient, and the quantification of the concentration in blood, serum and/or plasma, of said lipid mediators. In certain cases, this infection can lead to lung inflammation and/or uncontrolled production of cytokines In a third inventive aspect, the invention relates to the use of a compound of the present invention for the treatment of a patient diagnosed with an infection caused by a pathogen in the respiratory system, and which in certain cases can lead to pulmonary inflammation and/or uncontrolled production of cytokines, through the diagnostic method of the present invention.

### DETAILED DESCRIPTION OF THE INVENTION

The present invention describes the use of unsaturated analogs of docosanoic acid that are fundamentally characterized by having 5 or 6 double bonds in the carbon chain which is characteristic of this fatty acid of 22 carbon atoms, and the presence of a hydroxyl group at the C-14 position of the aforementioned chain, for the treatment of infections caused by a pathogen in the respiratory system. Compounds belonging to this family, in addition to the hydroxyl at the C-14 position, can have one or two additional hydroxyl groups, up to a total of three, also located in the carbon chain, specifically, at the C-4, C-7, and/or C-13 positions.

In a first embodiment, the invention relates to an unsaturated analog of docosanoic acid of formula (I), for the use mentioned above: 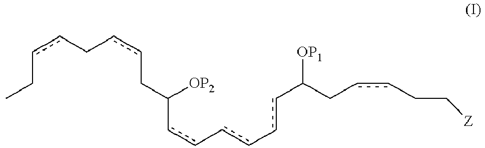
wherein each of P₁ and P₂ individually is a protecting group or a hydrogen atom;
wherein is -̅-̅-̅-̅-̅-̅ a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl;
each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy,-OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d},-[NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c}; each n, independently is an integer from 0 to 3;
each R^{d}, independently is a protecting group or R^{a};
or a pharmaceutically acceptable salt, tautomer, and/or solvate thereof;
for use in the treatment of an infection caused by a pathogen in the respiratory system. In certain aspects, P₁ and P₂ are both hydrogen atoms.

In another aspect, the double bonds at the 4, 10, 16 and 19 positions are each of cis (Z) configuration or the double bonds at the 4, 16 and 19 positions are each of the Z configuration.

In another embodiment, the double bonds at positions 8 and 10 are in the trans (E) configuration.

In yet another preferred embodiment, Z is -C (O) OR^{d} where R^{d} for Z is other than hydrogen.

In a preferred embodiment, the C-14 hydroxyl has S configuration.

In another preferred embodiment, the C-7 hydroxyl has R configuration.

In another embodiment, when the pathogen is not Covid-19, the following are expressly excluded:
7R,14S-Dihydroxy-4Z,8E,10E,12Z,16Z,1 9Z-docosahexaenoic acid;
7S,14S-Dihydroxy-4Z,8E,10Z,12E,16Z,19Z-docosahexaenoic; and
7S,14S-Dihydroxy-4Z,8E,10E,12Z,16Z,19Z-docosahexaenoic acid.

In a particular embodiment, the compound of formula (I) for the use mentioned above when the pathogen is Covid-19 is the compound of formula (Ia):

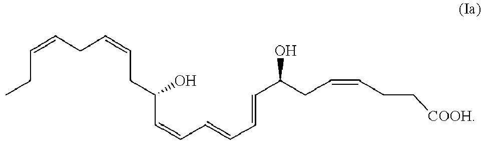

In a particular embodiment, the compound of formula (I) for the use mentioned above when the pathogen is Covid-19 is the compound of formula (Ib):

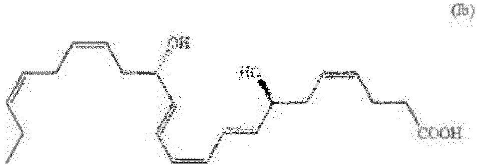

It should be understood that compounds (Ia) and (Ib) include all pharmaceutically acceptable salts, tautomers, and solvates thereof.

In a particular embodiment, the invention refers to an unsaturated analogue of docosanoic acid of formula (Ic) for the use mentioned above: 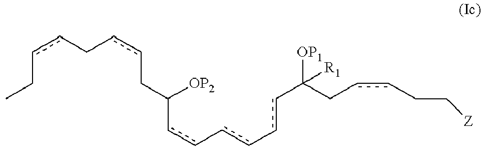 wherein P₁, P₂, -̅-̅-̅-̅-̅-̅, Z, R^{a}, R^{b}, R^{c}, R^{d} and n are as previously defined. R₁ is selected from (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl. In one aspect, R₁ is a methyl or ethyl group.

In other aspect, P₁ and P₂ are both hydrogen atoms. In another aspect, Z is -C(O)OR^{d} and R^{d} of Z is a hydrogen atom. In another embodiment, the double bonds at the 4, 10, 16 and 19 positions are each of Z configuration or the double bonds at the 4, 16 and 19 positions are each of the Z configuration or double bonds at 4, 16 and 19 positions are each of Z configuration. In other aspect, double bonds at 8 and 10 positions are in the trans (E) configuration. In other particular aspect, the hydroxyl at C-7 has an R configuration. In other particular aspect, the hydroxyl at C-14 has an S configuration.

In another particular embodiment, the invention relates to an unsaturated analog of docosanoic acid of formula (Id) for the use mentioned above: 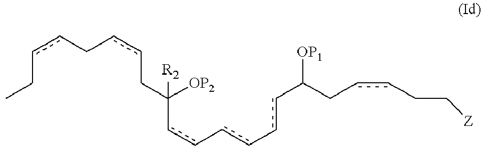 wherein P₁, P₂, -̅-̅-̅-̅-̅-̅, Z, R^{a}, R^{b}, R^{c}, R^{d} and n are as previously defined. R₂ is selected from (C1-C6) alkyl, (C3-C8) cycloalkyl, cyclohexyl, (C4-C11) cycloalkylalkyl, (C5-C10), phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl. In one aspect, R₂ is a methyl group.

In one aspect, P₁ and P₂ are both hydrogen atoms. In another aspect, Z is -C(O)OR^{d} and R^{d} of Z is a hydrogen atom. In another embodiment, the double bonds at the 4, 10, 16 and 19 positions are each of Z configuration or the double bonds at the 4, 10, 16 and 19 positions are each of the Z configuration or double bonds at the 4, 16, and 19 positions are each of the Z configuration. In still another embodiment, the hydroxyl at C-7 has an R configuration. In still yet another embodiment, the hydroxyl at C-14 has an S configuration.

In another aspect, the present invention relates to an unsaturated docosanoid acid analogue of formula (le) for the use mentioned above: 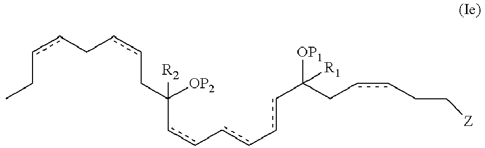 wherein P₁, P₂, -̅-̅-̅-̅-̅-̅, Z, R^{a}, R^{b}, R^{c}, R^{d}, R₁, R₂ and n are as previously defined. In a particular aspect, R1 and R2 are independently a methyl or ethyl group.

In one aspect, P₁ and P₂ are both hydrogen atoms. In still another aspect, Z is -C(O)OR^{d} and R^{d} of Z is a hydrogen atom. In another embodiment, the double bonds at the 4, 10, 16 and 19 positions are each of Z configuration or the double bonds at the 4, 16 and 19 positions are each of the Z configuration. In another embodiment, the double bonds at positions 8 and 10 are in the E configuration. In still another embodiment, the hydroxyl at C-7 has an R configuration. In still yet another embodiment, the hydroxyl at C-14 has S configuration.

In another aspect, the invention relates to an unsaturated docosanoic acid analogue of formula (II) for the use mentioned above: 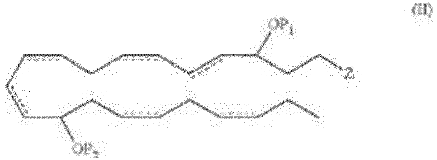
wherein each individually P₁ and P₂ is a protecting group or a hydrogen atom; wherein -̅-̅-̅-̅-̅-̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl;
wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy,-OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c}; each n, independently is an integer from 0 to 3;
each R^{d}, independently is a protecting group or R^{a};
or a pharmaceutically acceptable salt, tautomer, and/or solvate thereof.

In certain aspects, P₁ and P₂ are both hydrogen atoms.

In another embodiment, the double bonds at 7, 10, 16 and 19 positions are each of Z configuration. In another particular embodiment, the double bonds at 5 and 12 positions are each of E configuration.

In another aspect, the present invention provides an unsaturated docosanoic acid analogue of formula (Ila) for the use mentioned above: 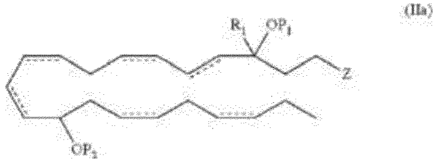 wherein P₁, P₂, -̅-̅-̅-̅-̅-̅, Z, R^{a}, R^{b}, R^{c}, R^{d}, R₁ and n are as previously defined. In a particular aspect, R₁ is a methyl or ethyl group.

In another aspect, P₁ and P₂ are both hydrogen atoms. In another aspect, Z is -C(O)OR^{d} and R^{d} of Z is a hydrogen atom. In still another aspect, the double bonds at the 7, 10, 16 and 19 positions are each of Z configuration. In still another aspect, the double bonds at the 5 and 12 positions are each of E configuration. In yet another aspect, the hydroxyl at C-14 has S configuration.

In another aspect, the present invention relates to an unsaturated docosanoic acid analogue of formula (IIb) for the use mentioned above: 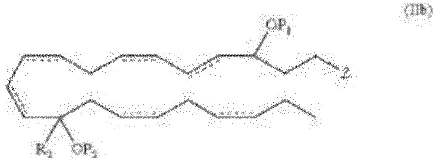 wherein P₁, P₂, -̅-̅-̅-̅-̅-̅, Z, R^{a}, R^{b}, R^{c}, R^{d}, R₂ and n are as previously defined. In a particular aspect, R₂ is a methyl or ethyl group.

In one aspect, P₁ and P₂ are both hydrogen atoms. In another aspect, Z is -C(O)OR^{d} and R^{d} of Z is a hydrogen atom. In still another aspect, the double bonds at the 7, 10, 16 and 19 positions are each of Z configuration. In still another aspect, the double bonds at the 5 and 12 positions are each of E configuration. In yet another aspect, the hydroxyl at C-14 has S configuration.

In another aspect, the present invention relates to an unsaturated docosanoic acid analogue of formula (IIc) for the use mentioned above: 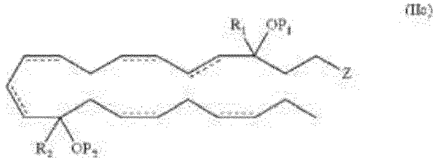 wherein P₁, P₂, -̅-̅-̅-̅-̅-̅, Z, R^{a}, R^{b}, R^{c}, R^{d}, R₁, R₂ and n are as previously defined. In a particular aspect, R₁ and R₂ are independently a methyl or ethyl group.

In one aspect, P₁ and P₂ are both hydrogen atoms. In another aspect, Z is -C(O)OR^{d} and R^{d} of Z is a hydrogen atom. In still another aspect, the double bonds at the 7, 10, 16 and 19 positions are each of Z configuration. In still another aspect, the double bonds at the 5 and 12 positions are each of E configuration. In yet another aspect, the hydroxyl at C-14 has S configuration.

In another aspect, the invention relates to an unsaturated docosanoic acid analogue of formula (III) for the use mentioned above: 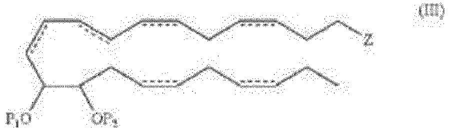
wherein each individually P₁ and P₂ is a protecting group or a hydrogen atom;
wherein -̅-̅-̅-̅-̅-̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl;
wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy,-OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d},-[NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c}; each n, independently is an integer from 0 to 3;
each R^{d}, independently is a protecting group or R^{a};
or a pharmaceutically acceptable salt, tautomer, and/or solvate thereof.

In certain aspects, P₁ and P₂ are both hydrogen atoms.

In certain preferred aspects, Z is -C(O)OR^{d} where R^{d} for Z is hydrogen.

In a preferred embodiment, the hydroxyl at C-14 has S configuration. In another preferred embodiment, the hydroxyl at C-7 has R configuration.

In another embodiment, the double bonds at 4, 7, 16 and 19 positions are each of Z configuration. In another particular embodiment, the double bonds at 9 and 11 positions are each of E configuration.

In another embodiment, when the pathogen is not Covid-19, 13R,14S-Dihydroxy-4Z, 7Z, 9E,11E,16Z,19Z-docosahexaenoic acid is expressly excluded.

In another particular embodiment, the invention relates to an unsaturated docosanoic acid analog of formula (Illa) for the use mentioned above: 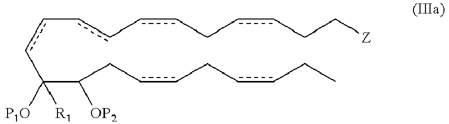 wherein P₁, P₂, -̅-̅-̅-̅-̅-̅, Z, R^{a}, R^{b}, R^{c}, R^{d}, R₁ and n are as previously defined. In a particular embodiment, R₁ is a methyl or ethyl group.

In one aspect, P₁ and P₂ are both hydrogen atoms. In another aspect, Z is -C(O)OR^{d} and R^{d} of Z is a hydrogen atom. In another embodiment, the double bonds at the 4, 7, 16 and 19 positions are each of Z configuration. In another particular embodiment, the double bonds at 9 and 11 positions are each in the E configuration.

In another particular embodiment, the invention relates to an unsaturated docosanoic acid analog of formula (Illb) for the use mentioned above: 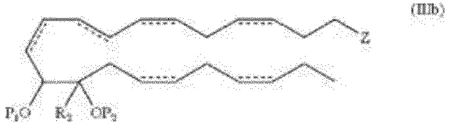 wherein P₁, P₂, -̅-̅-̅-̅-̅-̅, Z, R^{a}, R^{b}, R^{c}, R^{d}, R₂ and n are as previously defined. In a particular embodiment, R₂ is a methyl or ethyl group.

In one aspect, P₁ and P₂ are both hydrogen atoms. In another aspect, Z is -C(O)OR^{d} and R^{d} of Z is a hydrogen atom. In another embodiment, the double bonds at the 4, 7, 16 and 19 positions are each of Z configuration. In another particular embodiment, the double bonds at 9 and 11 positions are each in the E configuration.

In another particular embodiment, the invention relates to an unsaturated docosanoic acid analog of formula (IIIc) for the use mentioned above: 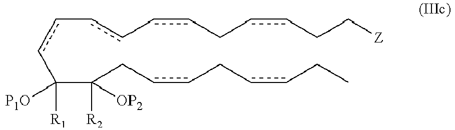 wherein P₁, P₂,-̅-̅-̅-̅-̅-̅, Z, R^{a}, R^{b}, R^{c}, R^{d}, R₁, R₂ and n are as previously defined. In a particular aspect, R₁ and R₂ are independently a methyl or ethyl group.

In one aspect, P₁ and P₂ are both hydrogen atoms. In another aspect, Z is -C(O)OR^{d} and R^{d} of Z is a hydrogen atom. In another embodiment, the double bonds at the 4, 7, 16 and 19 positions are each of Z configuration. In another particular embodiment, the double bonds at 9 and 11 positions are each in the E configuration.

In another particular embodiment, the invention relates to an unsaturated docosanoic acid analog of formula (IIId) for the use mentioned above: 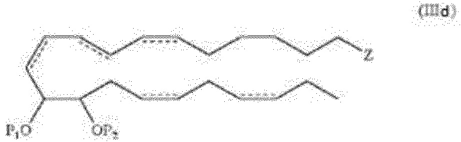 wherein P₁, P₂, -̅-̅-̅-̅-̅-̅, Z, R^{a}, R^{b}, R^{c}, R^{d}, and n are as previously defined. In one aspect, P₁ and P₂ are both hydrogen atoms. In another embodiment, the double bonds at the 7, 16 and 19 positions are each of Z configuration. In another particular embodiment, the double bonds at 9 and 11 positions are each in the E configuration.

In a particular embodiment, the invention relates to an unsaturated docosanoic acid analog of formula (IV) for the use mentioned above: 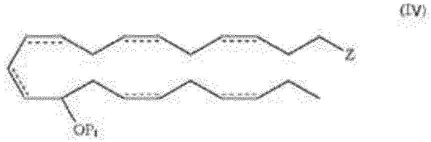
wherein P₁ is a protecting group or a hydrogen atom;
wherein -̅-̅-̅-̅-̅-̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy,-OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d},-[NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for the use in the treatment of an infection caused by a pathogen in the respiratory system.

In certain aspects, P₁ and P₂ are both hydrogen atoms. In certain preferred aspects, Z is -C(O)OR^{d} where R^{d} for Z is hydrogen. In another embodiment, the double bonds at 4, 7, 10, 16 and 19 positions are each of Z configuration. In another more particular embodiment, the double bonds at 12 position is of E configuration.

In another embodiment, when the pathogen is not Covid-19 are expressly excluded:
14-Hydroxy-4Z,7Z,10Z,12Z,16Z,19Z-docosahexaenoic acid;
14-Hydroxy-4Z,7Z,10Z,12E,16Z,19Z-docosahexaenoic acid; and
Methyl 14-hydroxy-4Z,7Z,10Z,12E,16Z,19Z-docosahexaenoate.

In a particular embodiment, the invention relates to an unsaturated docosanoic acid analog of formula (IVa) for the use mentioned above: 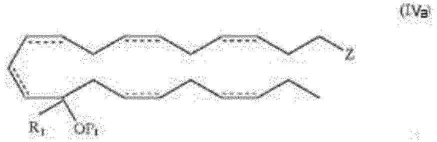 wherein P₁, -̅-̅-̅-̅-̅-̅, Z, R^{a}, R^{b}, R^{c}, R^{d}, R₁ and n are as previously defined. In one aspect, P₁ is hydrogen. In a particular embodiment, R₁ is a methyl or ethyl group. In another aspect, Z is -C(O)OR^{d} and R^{d} of Z is a hydrogen atom. In another embodiment, the double bonds at the 4, 7, 10, 16 and 19 positions are each of Z configuration. In another particular embodiment, the double bond at 12 position is in the E configuration.

In a particular embodiment, the invention relates to an unsaturated docosanoic acid analog of formula (IVb) for the use mentioned above: 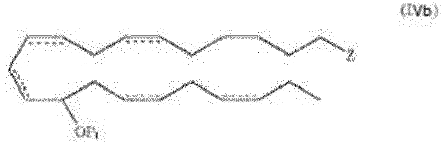 wherein P₁, -̅-̅-̅-̅-̅-̅, Z, R^{a}, R^{b}, R^{c}, R^{d} and n are as previously defined. In one aspect, P₁ is hydrogen. In another aspect, Z is -C(O)OR^{d}and R^{d} of Z is a hydrogen atom. In another embodiment, the double bonds at the 7, 10, 16 and 19 positions are each of Z configuration. In another particular embodiment, the double bond at 12 position is in the E configuration.

In a particular embodiment, the invention relates to an unsaturated docosanoic acid analog of formula (V) for the use mentioned above: 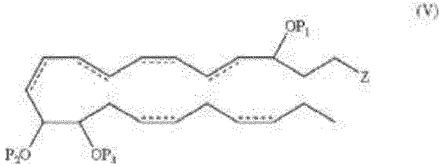 wherein each of P₁, P₂ and P₃ individually is a protecting group or a hydrogen atom and -̅-̅-̅-̅-̅-̅, Z, R^{a}, R^{b}, R^{c}, R^{d} and n are as previously defined.

In one aspect, P₁, P₂ and P₃ are all hydrogen.

In another aspect, the double bonds at the 7, 16 and 19 positions are each of Z configuration. In another embodiment, the double bonds at 5, 9 and 11 positions are all in the E configuration.

In a particular embodiment, the invention relates to an unsaturated docosanoic acid analog of formula (Va) for the use mentioned above: 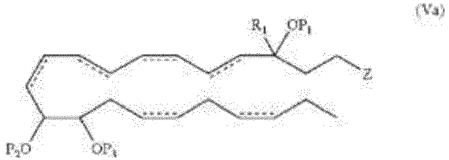 wherein P₁, P₂ and P₃, -̅-̅-̅-̅-̅-̅, Z, R^{a}, R^{b}, R^{c}, R^{d}, R₁ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₁ is a methyl or ethyl group.

In a particular embodiment, the invention relates to an unsaturated docosanoic acid analog of formula (Vb) for the use mentioned above: 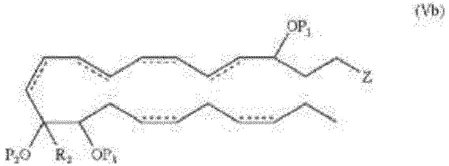 wherein P₁, P₂ and P₃, -̅-̅-̅-̅-̅-̅, Z, R^{a}, R^{b}, R^{c}, R^{d}, R₂ and n are as previously defined.

In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₂ is a methyl or ethyl group.

In a particular embodiment, the invention relates to an unsaturated docosanoic acid analog of formula (Vc) for the use mentioned above: 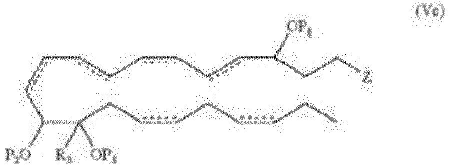 wherein P₁, P₂ and P₃, -̅-̅-̅-̅-̅-̅, Z, R^{a}, R^{b}, R^{c}, R^{d}, R₃ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₃ is a methyl or ethyl group.

In a particular embodiment, the invention relates to an unsaturated docosanoic acid analog of formula (Vd) for the use mentioned above: 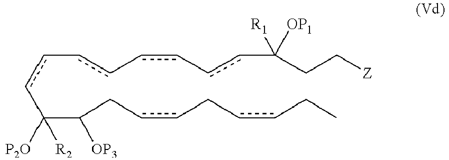 wherein P₁, P₂ and P₃, -, Z, R^{a}, R^{b}, R^{c}, R^{d}, R₁, R₂ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₁ and R₂ are independently a methyl or ethyl group.

In a particular embodiment, the invention relates to an unsaturated docosanoic acid analog of formula (Ve) for the use mentioned above: 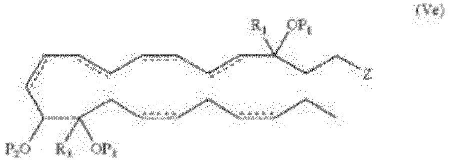 wherein P₁, P₂ and P₃, -̅-̅-̅-̅-̅-̅, Z, R^{a}, R^{b}, R^{c}, R^{d}, R₁, R₃ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₁ and R₃ are independently a methyl or ethyl group.

In a particular embodiment, the invention relates to an unsaturated docosanoic acid analog of formula (Vf) for the use mentioned above: 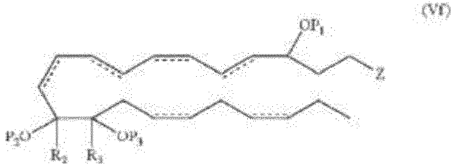 wherein P₁, P₂ and P₃, -̅-̅-̅-̅-̅-̅, Z, R^{a}, R^{b}, R^{c}, R^{d}, R₂, R₃ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₂ and R₃ are independently a methyl or ethyl group.

In a particular embodiment, the invention relates to an unsaturated docosanoic acid analog of formula (Vg) for the use mentioned above: 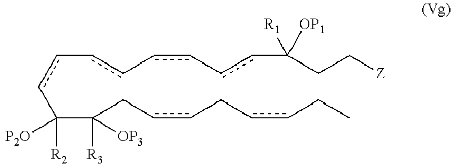 wherein P₁, P₂ and P₃, -̅-̅-̅-̅-̅-̅, Z, R^{a}, R^{b}, R^{c}, R^{d}, R₁, R₂, R₃ and n are as previously defined. In one aspect, P₁, P₂ and P₃ are all hydrogen. In another aspect, the double bonds at the 7, 16 and 19 positions are each of Z configuration. In another aspect, the double bonds at 5, 9 and 11 positions are all in the E configuration. In other aspect, R₁, R₂ and R₃ are independently a methyl or ethyl group.

In a particular embodiment, the invention relates to an unsaturated docosanoic acid analog of formula (VI) for the use mentioned above: 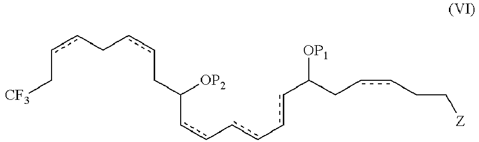 wherein P₁, P₂, -̅-̅-̅-̅-̅-̅, Z, R^{a}, R^{b}, R^{c}, R^{d} and n are as previously defined.

In one aspect, P₁ and P₂ are both hydrogen. In another aspect, Z is -C(O)OR^{d} and R^{d} of Z is a hydrogen atom. In another aspect, the double bonds at the 4, 12, 16 and 19 positions are each of Z configuration. In another embodiment, the double bonds at 8 and 10 positions are all in the E configuration. In another aspect, the hydroxyl at position 7 is in the R configuration. In another aspect, the hydroxyl at position 14 is in the S configuration.

In a particular embodiment, the invention relates to an unsaturated docosanoic acid analog of formula (IVa) for the use mentioned above: 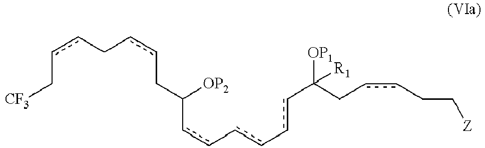 wherein P₁, P₂, -̅-̅-̅-̅-̅-̅, Z, R^{a}, R^{b}, R^{c}, R^{d}, R₁ and n are as previously defined. In one aspect, P₁ and P₂ are both hydrogen. In another aspect, Z is -C(O)OR^{d}and R^{d} of Z is a hydrogen atom. In other aspect, R₁ is a methyl or ethyl group. In another aspect, the double bonds at the 4, 12, 16 and 19 positions are each of Z configuration. In another embodiment, the double bonds at 8 and 10 positions are all in the E configuration. In another aspect, the hydroxyl at 7 position is in the R configuration. In another aspect, the hydroxyl at 14 position is in the S configuration.

In a particular embodiment, the invention relates to an unsaturated docosanoic acid analog of formula (IVb) for the use mentioned above: 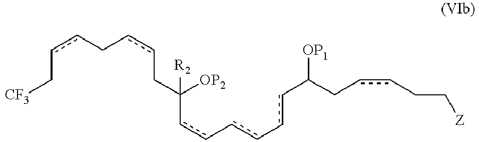 wherein P₁, P₂, -̅-̅-̅-̅-̅-̅, Z, R^{a}, R^{b}, R^{c}, R^{d}, R₂ and n are as previously defined. In one aspect, P₁ and P₂ are both hydrogen. In another aspect, Z is -C(O)OR^{d}and R^{d} of Z is a hydrogen atom. In other aspect, R₂ is a methyl or ethyl group. In another aspect, the double bonds at the 4, 12, 16 and 19 positions are each of Z configuration. In another embodiment, the double bonds at 8 and 10 positions are all in the E configuration. In another aspect, the hydroxyl at 7 position is in the R configuration. In another aspect, the hydroxyl at 14 position is in the S configuration.

In a particular embodiment, the invention relates to an unsaturated docosanoic acid analog of formula (IVc) for the use mentioned above: 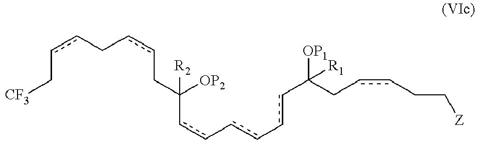 wherein P₁, P₂, -̅-̅-̅-̅-̅-̅, Z, R^{a}, R^{b}, R^{c}, R^{d}, R₁, R₂ and n are as previously defined. In one aspect, P₁ and P₂ are both hydrogen. In another aspect, Z is -C(O)OR^{d}and R^{d} of Z is a hydrogen atom. In other aspect, R₁ and R₂ are independently a methyl or ethyl group. In another aspect, the double bonds at the 4, 12, 16 and 19 positions are each of Z configuration. In another embodiment, the double bonds at 8 and 10 positions are all in the E configuration. In another aspect, the hydroxyl at 7 position is in the R configuration. In another aspect, the hydroxyl at 14 position is in the S configuration.

In a particular embodiment, the invention relates to an unsaturated docosanoic acid analog of formula (IVd) for the use mentioned above: 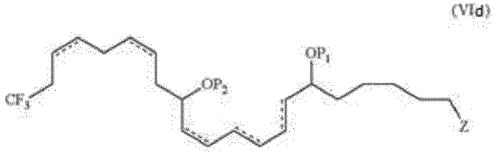 wherein P₁, P₂, -̅-̅-̅-̅-̅-̅, Z, R^{a}, R^{b}, R^{c}, R^{d} and n are as previously defined. In one aspect, P₁ and P₂ are all hydrogen. In another aspect, Z is -C(O)OR^{d} and R^{d} of Z is a hydrogen atom. In another aspect, the double bonds at the 12, 16 and 19 positions are each of Z configuration. In another aspect, the double bonds at 8 and 10 positions are all in the E configuration. In another aspect, the hydroxyl at 7 position is in the R configuration. In another aspect, the hydroxyl at 14 position is in the S configuration.

In a particular embodiment, the invention relates to an unsaturated docosanoic acid analog of formula (VII) for the use mentioned above: 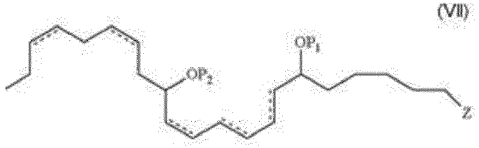 wherein P₁, P₂, -̅-̅-̅-̅-̅-̅, Z, R^{a}, R^{b}, R^{c}, R^{d} and n are as previously defined. In another aspect, P₁ and P₂ are both hydrogen. In another aspect, the double bonds at the 12, 16 and 19 positions are each of cis (Z) configuration or double bonds at 16 and 19 positions are each of cis (Z) configuration. In another particular aspect, the double bonds at 8 and 10 positions are all in the E configuration. In another aspect, the hydroxyl at C-7 is in the R configuration. In another aspect, the hydroxyl at C-14 is in the S configuration.

In another particular aspect, when the pathogen is not Covid-19, then 7,14-Dihydroxy-8E,10E,12Z,16Z,19Z-eicosapentaenoic acid is expressly excluded.

In another particular embodiment, the present invention provides the use of pharmaceutical compositions comprising one or more compounds of the invention, with or without other active pharmaceutical ingredients, in mixture with a pharmaceutically acceptable carrier, for the treatment of an infection caused by a pathogen in the respiratory system. Such a composition can be administered according to the methods of the present invention.

In another embodiment, the present invention is based on the use of a compound of the invention, or a composition (for example, a dietary supplement or a medical food) or a pharmaceutical composition comprising a compound of the present invention, where it is expressly excluded the compounds of formula (IV) 14-Hydroxy-4Z,7Z,10Z,12E,16Z,19Z-docosahexaenoic acid, and Ethyl 14-hydroxy-4Z,7Z,10Z,12E,16Z,19Z-docosahexaenoate, for the treatment or prevention of an infection with cytokine release syndrome or cytokine storm syndrome in a mammal. The use or treatment involves the administration of a prophylactic or therapeutically effective amount of at least one compound of the invention, a composition, or a pharmaceutical composition thereof.

In another more preferred embodiment, the compound of the invention, composition (for example, a dietary supplement or a medical food) or pharmaceutical composition comprising at least one compound of the present invention can be used to treat or prevent an infection selected from viral pneumonia, bacterial pneumonia, or atypical pneumonia.

In another embodiment, the compound of the invention, composition (for example, a dietary supplement or a medical food) or pharmaceutical composition comprising at least one compound of the present invention can be used to treat or prevent an infection caused by a pathogen in the respiratory system and that causes pulmonary inflammation.

In another more preferred embodiment, the compound of the invention, composition (for example, a dietary supplement or a medical food) or pharmaceutical composition comprising at least one compound of the present invention can be used to treat or prevent an infection selected from pneumonia, bacterial pneumonia, viral pneumonia, atypical pneumonia, Graft versus host disease (GVHD), coronavirus disease 2019 (COVID-19), viral load reduction of Covid-19, SARS-CoV1, Ebola, avian Flu, smallpox, or the common flu, by administering to an individual in need thereof, an effective amount of any one of the compounds, compositions, or pharmaceutical compositions described herein.

In another embodiment, the compounds of the invention, compositions (e.g., a dietary supplement or a medical food) or pharmaceutical compositions comprising at least one compound of the present invention can be used to treat or prevent a viral infection.

In another more preferred embodiment, the compounds of the invention, compositions (for example, a dietary supplement or a medical food) or pharmaceutical compositions comprising at least one compound of the present invention can be used to treat or prevent a viral pneumonia.

In another even more preferred embodiment, the compounds of the invention, compositions (for example, a dietary supplement or a medical food) or pharmaceutical compositions comprising at least one compound of the present invention can be used to treat or prevent a disease selected from between COVID-19, SARS-CoV1, sepsis, Ebola, bird flu, smallpox, or the common flu.

In another still more preferred embodiment, the compounds of the invention, compositions (for example, a dietary supplement or a medical food) or pharmaceutical compositions comprising at least one compound of the present invention can be used to treat or prevent COVID-19.

In another embodiment, the compounds of the invention, compositions (for example, a dietary supplement or a medical food) or pharmaceutical compositions comprising at least one compound of the present invention can be used to treat or prevent an infection leading to the acute respiratory distress syndrome (ARDS), severe acute respiratory syndrome (SARS), systemic inflammatory response syndrome (SIRS), severe fever with thrombocytopenia syndrome (SFTS), or pancreatitis.

In another more preferred embodiment, the compounds of the invention, compositions (for example, a dietary supplement or a medical food) or pharmaceutical compositions comprising at least one compound of the present invention can be used to treat or prevent an infection that occurs with acute respiratory distress syndrome (ARDS), or severe acute respiratory syndrome (SARS).

In another embodiment, the compounds of the invention, compositions (e.g., a dietary supplement or a medical food) or pharmaceutical compositions comprising at least one compound of the present invention can be used to treat or prevent an infection leading to pancreatitis.

In US10568858, Serhan C.N. *et al*., disclose compositions comprising at least one compound of the present invention, as for example, the compound of formula (IV) 14-HDHA or 14-hydroxy-4Z,7Z,10Z,12E,16Z,19Z-docosahexaenoic acid, and other compounds such as 17-HDHA, 18-HEPE, 4-HDHA, and 10-HDHA both in their free form as ethyl ester, triglyceride, diglyceride, monoglyceride or mixtures thereof, for the treatment of diseases that present with inflammation. In this patent, the synergistic effect of a mixture of several polyunsaturated and hydroxylated long chain fatty acids is shown. Compositions such as those described in US10568858 are marketed under the name Lipinova^{®}.

In another embodiment, the present invention refers to the use, as mentioned above, of any one of the pharmaceutical compositions, comprising one or more compounds of the present invention, as defined by formulas: (I), (Ia ), (Ib), (Ic), (Id), (Ie), (II), (IIa), (IIb), (IIc), (III), (IIIa), (IIIb), (IIIc), (IIId), (IV), (IVa), (IVb), (V), (Va), (Vb), (Vc), (Vd), (Ve), (Vf), (Vg), (VI ), (VIa), (Vlb), (VIc), (VId), and (VII), and the compounds: 17-HDHA (17-hydroxy-4Z, 7Z,10Z,13Z,15E,19Z-docosahexaenoic acid) and 18-HEPE (18-hydroxy-5Z,8Z,11Z, 14Z,16E-eicosapentaenoic acid).

In another more preferred embodiment, the present invention relates to the use, as mentioned above, of a pharmaceutical composition, comprising at least one compound of formula (IV), and the compounds 17-HDHA and 18-HEPE.

In another more preferred embodiment, the present invention relates to the use, as mentioned above, of a pharmaceutical composition comprising 14-HDHA, 17-HDHA and 18-HEPE.

In another more preferred embodiment, the present invention refers to the use, as mentioned above, of a dietary supplement, or a medical food comprising at least one of the compounds of the invention defined according to the formulas: (I), (Ia), (Ib), (Ic), (Id), (le), (If), (II), (IIa), (IIb), (IIc), (III), (IIIa), (IIIb), (IIIc), (IIId), (IV), (IVa), (IVb), (V), (Va), (Vb), (Vc), (Vd), (Ve), (Vf), (Vg), (VI), (Vla), (Vlb), (VIc), or (VId), and the compounds 17-HDHA and 18-HEPE.

In a more preferred embodiment, the present invention relates to the use, as mentioned above, of a dietary supplement or a medical food comprising at least one compound of formula (IV), and the compounds 17-HDHA and 18-HEPE.

In an even more preferred embodiment, the present invention relates to the use, as mentioned above, of a dietary supplement comprising 14-HDHA, 17-HDHA and 18-HEPE.

In another still more preferred embodiment, 14-HDHA is present in the pharmaceutical composition, nutritional composition, medical food, or dietary supplement, in an amount from 0.0002% by weight to 10% by weight, for example, 0.0002, 0,0004, 0,0006, 0,0008, 0,001, 0,002, 0,003, 0,004, 0,005, 0,006, 0,007, 0,008, 0,009, 0,01, 0,02, 0,03, 0,04, 0,05, 0,06, 0,07, 0,08, 0,09, 0,1, 0,12, 0,14, 0,16, 0,18, 0,2 % by weight, 0,22 % by weight, 0,24 % by weight, 0,26 % by weight, 0,28 % by weight, 0,3 % by weight, 0,32 % by weight, 0,34 % by weight, 0,36 % by weight, 0,38 % by weight, 0,4 % by weight, 0,42 % by weight, 0,44 % by weight, 0,46 % by weight, 0,48 % by weight, 0,5 % by weight, 0,52 % by weight, 0,54 % by weight, 0,56 % by weight, 0,58 % by weight, 0,6 % by weight, 0,62 % by weight, 0,64 % by weight, 0,66 % by weight, 0,68 % by weight, 0,7 % by weight, 0,72 % by weight, 0,74 % by weight, 0,76 % by weight, 0,78 % by weight, 0,8 % by weight, 0,82 % by weight, 0,84 % by weight, 0,86 % by weight, 0,88 % by weight, 0,9 % by weight, 0,92 % by weight, 0,94 % by weight, 0,96 % by weight, 0,98 % by weight, 1,0 % by weight, 1,5 % by weight, 2,0 % by weight, 2,5 % by weight, 3,0 % by weight, 3.5 % by weight, 4,0 % by weight, 4,5 % by weight, 5,0 % by weight, 5,5 % by weight, 6,0 % by weight, 6,5 % by weight, 7,0 % by weight, 7,5 % by weight, 8,0 % by weight, 8,5 % by weight, 9,0 % by weight, 9,5 % by weight, or 10,0 % by weight.

In a preferred embodiment, 14-HDHA is present in an amount between 0.008 % by weight and 10.0 % by weight.

In another still more preferred embodiment, 17-HDHA is present in the pharmaceutical composition, nutritional composition, medical food, or dietary supplement, in an amount of 0.0002% by weight to 10% by weight, for example, 0.0002, 0.0004, 0.0006, 0.0008, 0.001, 0.002, 0.003, 0.004, 0.005, 0.006, 0.007, 0.008, 0.009, 0.01, 0.02, 0.03, 0.04, 0.05 , 0.06, 0.07, 0.08, 0.09, 0.1, 0.12, 0.14, 0.16, 0.18, 0.2% by weight, 0.22% by weight , 0.24% by weight, 0.26% by weight, 0.28% by weight, 0.3% by weight, 0.32% by weight, 0.34% by weight, 0.36% by weight, 0.38% by weight, 0.4% by weight, 0.42% by weight, 0.44% by weight, 0.46% by weight, 0.48% by weight, 0.5% by weight, 0, 52% by weight, 0.54% by weight, 0.56% by weight, 0.58% by weight, 0.6% by weight, 0.62% by weight, 0.64% by weight, 0, 66% by weight, 0.68% by weight, 0.7% by weight, 0.72% by weight, 0.74% by weight, 0.76% by weight, 0.78% by weight, 0.8 % by weight, 0.82% by weight, 0.84% by weight, 0.86% by weight, 0.88% by weight, 0.9% by weight, 0.92% by weight, 0.94% by weight, 0.96% by weight, 0.98% by weight, 1.0% by weight, 1.5% by weight, 2.0% by weight, 2.5% by weight, 3.0% by weight, 3.5% by weight, 4.0% by weight, 4.5% by weight, 5.0% by weight, 5.5% by weight, 6.0% by weight, 6.5% by weight, 7.0% by weight, 7.5% by weight, 8.0% by weight, 8.5% by weight, 9.0% by weight, 9.5% by weight, or 10.0% by weight.

In another preferred embodiment, 17-HDHA is present in an amount between 0.009% by weight and 10.0% by weight.

In another still more preferred embodiment, 18-HEPE is present in the pharmaceutical composition, nutritional composition, medical food, or dietary supplement, in an amount from 0.0002 % by weight to 10 % by weight, for example, 0.0002, 0.0004, 0.0006, 0.0008, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.12, 0.14, 0.16, 0.18, 0.2% by weight, 0.22% by weight, 0.24% by weight, 0.26% by weight, 0.28% by weight, 0.3% by weight, 0.32% by weight, 0.34% by weight, 0.36% by weight, 0.38% by weight, 0.4% by weight, 0.42% by weight, 0.44% by weight, 0.46% by weight, 0.48% by weight, 0.5% by weight, 0.52% by weight, 0.54% by weight, 0.56% by weight, 0.58% by weight, 0.6% by weight, 0.62% by weight, 0.64% by weight, 0.66% by weight, 0.68% by weight, 0.7% by weight, 0.72% by weight, 0.74% by weight, 0.76% by weight, 0.78% by weight, 0.8 % by weight, 0.82 % by weight, 0.84% by weight, 0.86 % by weight, 0.88 % by weight, 0, 9% by weight, 0.92% by weight, 0.94% by weight, 0.96% by weight, 0.98% by weight, 1% by weight, 1.5% by weight, 2.0% by weight, 2.5% by weight, 3.0% by weight, 3.5% by weight, 4.0% by weight, 4.5% by weight, 5.0% by weight, 5.5% by weight, 6.0% by weight, 6.5% by weight, 7.0% by weight, 7.5% by weight, 8.0% by weight, 8.5% by weight, 9.0% by weight, 9.5% by weight, or 10.0% by weight.

In another preferred embodiment, 18-HEPE is present in an amount between 0.02% by weight and 10.0% by weight.

In another more preferred embodiment, 17-HDHA is present in an amount between 0.009% by weight and 10.0% by weight, 18-HEPE is present in an amount between 0.01% by weight and 10.0% by weight, and 14-HDHA is present in an amount between 0.008% by weight and 10.0% by weight.

In another more preferred embodiment, 17-HDHA is present in an amount between 0.01% by weight and 10.0% by weight, 18-HEPE is present in an amount between 0.01% by weight and 10.0% in weight, and 14-HDHA is present in an amount between 0.008% by weight and 10.0% by weight.

It is understood in the present invention that the compounds of the present invention, especially the compound 14-HDHA, and the compounds 17-HDHA and 18-HEPE can be found in a pharmaceutical composition, in a dietary supplement or in a medical food in the free form (free acid), as esters such as ethyl esters, triglycerides, diglycerides, monoglycerides, sphingolipids, phospholipids, or as mixtures thereof.

Dietary supplements or medical foods of the present invention can include one or more active ingredients, such as aspirin, curcumin, polyphenols, lutein, astaxanthin, and/or one or more vitamins selected from vitamin D, vitamin A, vitamin E and/or vitamin K.

A second inventive aspect, the present invention comprises a method of diagnosing and/or monitoring the condition of a patient suffering from a disease caused by the presence of a pathogen in the respiratory system based on the identification of pro-resolutive lipid mediators and quantification of its concentration, in the blood, plasma, and/or serum of said patient, which are relevant in said disease.

In the human body, more than 70 lipid mediators related to inflammation have been identified that show a pro-inflammatory action (such as prostaglandins, thromboxanes, and leukotrienes) and/or pro-resolutive action (a wide list of this type of mediators can be found in WO201317006).

The concentration of lipid mediators, both pro-inflammatory and pro-resolutive, varies in a subject depending on whether he or she suffers from a pathology, disease, infection, insult, or aggression with respect to a subject considered healthy, that is, a subject who lacks of a pathology, disease, infection, insult, or any aggression.

In a preferred embodiment, the diagnostic method of the present invention refers to the identification of a cluster of five lipid mediators with pro-resolutive action formed by: RvE1 (5S,12R,18R-Trihydroxy-6Z,8E,10E,14Z,16E-eicosapentaenoic acid), RvD2 (7S,16R, 17S-Trihydroxy-4Z,8E,10Z,12E,14E,19Z-docosahexaenoic acid), RvD4 (4S,5,17S-Trihydroxy-6E,8E,10E,13E,15Z,19Z-docosahexaenoic acid), MaR1 (7R,14S-Dihydroxy-4Z,8E,10E,12Z,16Z,19Z-docosahexaenoic acid), and PDX (10S,17S-Dihydroxy-4Z,7Z, 11E,13Z,15E,19Z-docosahexaenoic acid), whose variations are relevant.

Surprisingly, by identifying and measuring the concentration of a high number of lipid mediators, through the analysis of the blood of a series of individuals, it has been found that variations in concentration of the aforementioned five lipid mediators in blood, plasma and/or serum, are related to the condition of a subject in relation to one of the diseases described in the present invention. In this way, it can be determined whether a subject is healthy or suffers from a disease caused by a pathogen in the respiratory system by analyzing the aforementioned pro-resolutive lipid mediators in blood, plasma, and/or serum. Furthermore, obtaining and analyzing the values in blood, plasma, and/or serum of the aforementioned lipid mediators allow monitoring the evolution of a patient suffering from a disease such as those described in the present invention.

Examples of methods for obtaining concentration values of lipid mediators of the present invention in blood, plasma, and/or serum can be found in WO2018098244, Serhan C. N., et al.*,* and/or WO2019057756A1, Dalli J., et al.

In a preferred embodiment, the cluster of pro-resolutive lipid mediators is composed of RvE1, RvD2, MaR1, and PDX.

In a preferred embodiment, the set of pro-resolution lipid mediators is composed of RvD2, MaR1, and PDX.

In a more preferred embodiment, the set of pro-resolution lipid mediators is composed of MaR1, and PDX.

In a more preferred embodiment, the diagnostic method and/or monitoring a patient suffering from a disease as mentioned above, is carried out by identifying and quantifying MaR1.

In a preferred embodiment, the measurement of the concentration values of the pro-resolutive lipid mediators is carried out in plasma.

In another preferred embodiment, the measurement of the concentration values of the pro-resolutive lipid mediators is carried out in serum.

In another preferred embodiment, the measurement of the concentration values of the pro-resolutive lipid mediators is carried out in tissue and/or fluids of the respiratory system.

In another more preferred embodiment, measurement of the concentration values of the pro-resolutive lipid mediators is carried out in plasma and in serum.

In another preferred embodiment, measurement of the concentration values of the pro-resolutive lipid mediators is carried out in serum, where the blood sample is treated with an adenosine inhibitor, as for example, adenosine deaminase (ADA), caffeine (1,3,7-trimethylxanthine or 3,7-dihydro-1,3,7-trimethyl-1H-purine-2,6-dione), 8-(3-chlorostyril) caffeine, 2-phenylaminoadenosine, 2-p-2 -carboxyethylphenylamino-5'-N-ethylcarboxamido-adenosine, 5-N-ethylcarboxamido-adenosine, 5'-N-cyclopropyladenosine, 5'N-methylcarboxamidoadenosine, PD-1259444, 1,3-dipropyl-phenylxanthine, or 4-{2-[7-amino-2-(2-furyl)[1,2,4]-triazolo[2,3-a]-[1,3,5]-triazin-5-ylamino]ethyl} phenol, also known as ZM 241385.

In a more preferred embodiment, in addition to the identification and measurement of the concentration values of the pro-resolutive lipid mediators RvE1, RvD2, RvD4, MaR1, and PDX, a cluster of so-called pro-inflammatory lipid mediators has also been identified. by: PGD2 (Prostaglandin D2, or 9α,15S-dihydroxy-11-oxo-prosta-5Z,13E-diene-1-oic acid) and LTB4 (Leukotriene B4 or 5S,12R-dihydroxy-6Z,8E,10E,14Z-eicosatetraenoic acid), whose identification and quantification of its concentration in blood, plasma, and/or serum allows the obtaining of additional information that complements or improves the diagnosis and/or follow-up of a subject suffering from one of the diseases described in the present invention.

In an even more preferred embodiment, identification and measurement of the concentration values of the pro-resolutive lipid mediators RvD2, MaR1, and PDX, and the pro-inflammatory mediator LTB4, are carried out.

In a preferred embodiment, measurement of the concentration values of the pro-inflammatory mediators is carried out in plasma.

In another preferred embodiment, measurement of the concentration values of the pro-inflammatory mediators is carried out in serum.

In another more preferred embodiment, measurement of the concentration values of the pro-inflammatory lipid mediators is carried out in plasma and in serum.

In another preferred embodiment, measurement of the concentration values of the pro-inflammatory lipid mediators is carried out in serum, where the blood sample is treated with an adenosine inhibitor, as for example, adenosine deaminase (ADA), caffeine (1,3,7-trimethylxanthine or 3,7-dihydro-1,3,7-trimethyl-1H-purine-2,6-dione), 8-(3-chlorostyril) caffeine, 2-phenylaminoadenosine, 2-p-2-carboxyethylphenylamino-5'-N-ethylcarboxamido-adenosine, 5-N-ethylcarboxamido-adenosine, 5'-N-cyclopropyladenosine, 5'N-methylcarboxamidoadenosine, PD-1259444, 1,3-dipropylphenylxanthine, or 4-{2-[7-amino-2-(2-furyl)[1,2,4]-triazolo[2,3-a]-[1,3,5]-triazin-5-ylamino]ethyl} phenol, the latter it is also known as ZM 241385.

In another preferred embodiment, measurement of the concentration values of the pro-inflammatory lipid mediators is carried out in serum, where the blood sample is treated with ADA.

In another preferred embodiment, measurement of the concentration values of the pro-inflammatory lipid mediators is carried out in serum, where the blood sample is treated with caffeine.

The authors of the present invention have found that serious or very serious patients with diseases caused by the presence of a pathogen in the respiratory system and that, in certain cases, they may present with uncontrolled production of cytokines, have altered levels of lipid mediators compared to healthy subjects. Thus, the so-called pro-resolutive lipid mediators are found at lower levels than the values established for healthy individuals, while the so-called pro-inflammatory lipid mediators are at higher levels than those found in healthy individuals.

In a preferred embodiment, the diagnostic and/or follow-up method allows a positive result, that is, a subject suffers from any one of those described in the present invention when a decrease of at least 30% is observed, with respect to a healthy individual, of the concentration in blood, plasma and/or serum of each one of the components of the cluster of pro-resolutive lipid mediators.

In a more preferred embodiment, the decrease in each of the components of the cluster of pro-resolutive lipid mediators is at least 40%, with respect to a healthy individual.

In an even more preferred embodiment, the decrease in each of the components of the cluster of pro-resolutive lipid mediators is at least 50%, with respect to a healthy individual.

In an even more preferred embodiment, the decrease in each of the components of the cluster of pro-resolutive lipid mediators is at least 60%, with respect to a healthy individual.

In an even more preferred embodiment, the decrease in each of the components of the cluster of pro-resolutive lipid mediators is at least 70%, with respect to a healthy individual.

In an even more preferred embodiment, the decrease in each of the components of the cluster of pro-resolutive lipid mediators is at least 80%, with respect to a healthy individual.

In an even more preferred embodiment, the decrease in each of the components of the cluster of pro-resolutive lipid mediators is at least 90%, with respect to a healthy individual.

In a preferred embodiment, the decrease in each of the components of the cluster of pro-resolutive lipid mediators is at least 30%, 35%, 40%, 45%, 50%, 55%, 60%, 65%, 70%, 75%, 80%, 85%, 90%, 95%, or 100%, while the increase in PGD2 is at least 200%, 250%, 300%, 350%, 400%, 450%, and the increase in LTB4 is at least 100%, 150%, 200%, 250%, with respect to a healthy individual.

In a more preferred embodiment, the decrease in each of the components of the cluster of pro-resolutive lipid mediators is at least 40%, and an increase of at least 200% in each of the pro-inflammatory mediators, PGD2 and LTB4, with respect to a healthy individual. In a more preferred embodiment, the decrease in each of the components of the cluster of pro-resolutive lipid mediators is at least 40%, and an increase in pro-inflammatory mediators of at least 300% of PGD2, and of, at least 200% of LTB4, with respect to a healthy individual.

In an even more preferred embodiment, the decrease in each of the components of the cluster of pro-resolutive lipid mediators is at least 40%, and an increase in pro-inflammatory mediators of at least 400% of PGD2, and of at least 200% of LTB4, with respect to a healthy individual.

In an even more preferred embodiment, the increase in pro-inflammatory mediator LTB4 is at least 100%, with respect to a healthy individual.

In a preferred embodiment, the method of diagnosing and/or monitoring a disease caused by the presence of a pathogen in the respiratory system is a disease that also occurs with cytokine release syndrome or cytokine storm syndrome in a mammal.

In another more preferred embodiment, the method of diagnosing and/or monitoring a disease caused by the presence of a pathogen in the respiratory system where the disease is selected from: pneumonia, bacterial pneumonia, viral pneumonia, atypical pneumonia, Graft versus host disease (GVHD), coronavirus disease 2019 (COVID-19), SARS-CoV1, acute respiratory distress syndrome (ARDS), severe acute respiratory Syndrome (SARS), sepsis, Ebola, avian flu, smallpox, systemic inflammatory response syndrome (SIRS), severe fever with thrombocytopenia syndrome (SFTS), common flu, and pancreatitis.

In another more preferred embodiment, the method of diagnosing and/or monitoring a disease caused by the presence of a pathogen in the respiratory system where the disease is selected from: viral pneumonia, bacterial pneumonia, and atypical pneumonia. In another even more preferred embodiment, the method of diagnosing and/or monitoring a disease caused by the presence of a pathogen in the respiratory system is viral pneumonia.

In another more preferred embodiment, the method of diagnosing and/or monitoring a disease caused by the presence of a pathogen in the respiratory system is selected from: COVID-19, SARS-CoV1, sepsis, Ebola, avian flu, smallpox, and common flu.

In another even more preferred embodiment, the method of diagnosis and/or monitoring of a disease caused by the presence of a pathogen in the respiratory system is Covid-19.

In another embodiment, the method of diagnosing and/or monitoring a disease caused by the presence of a pathogen in the respiratory system is selected from: acute respiratory distress syndrome (ARDS), severe acute respiratory syndrome (SARS), systemic inflammatory response syndrome (SIRS), severe fever with thrombocytopenia syndrome (SFTS), and pancreatitis.

In another more preferred embodiment, the method of diagnosing and/or monitoring a disease caused by the presence of a pathogen in the respiratory system is selected from: acute respiratory distress syndrome (ARDS), and severe acute respiratory syndrome (SARS).

In another embodiment, the disease related to the diagnostic and/or monitoring method of the present invention is pancreatitis.

In another embodiment, the disease related to the diagnostic and/or monitoring method of the present invention is a viral infection.

In a third inventive aspect, the invention relates to the use of a compound, a composition (as for example, a dietary supplement or a medical food), or a pharmaceutical composition, of the present invention for the treatment of a patient diagnosed through the diagnostic method of the present invention.

Additional features and advantages of the invention will become more apparent from the following detailed description and claims.

While multiple embodiments are described, still other embodiments of the present invention will be apparent to those skilled in the art from the following detailed description. As will be apparent, the invention is capable of modification in several obvious respects, all without departing from the spirit and scope of the present invention. Accordingly, the detailed descriptions should be considered illustrative and not restrictive in nature.

In the specification and claims, the terms "includes" and "comprises" are open ended terms and should be construed to mean "includes but is not limited to." These terms encompass the more restrictive terms "consists essentially of" and "consists of".

It should be noted that, as used herein and in the appended claims, the singular forms "a", "an" and "the" include the plural reference unless the context clearly dictates otherwise. Also, the terms "a" (or "an"), "one or more" and "at least one" can be used interchangeably herein. It is also to be noted that the terms "comprises", "includes" "characterized by" and "has" can be used interchangeably.

Unless defined otherwise, all technical and scientific terms used herein have the same meanings as are commonly understood by one of ordinary skill in the art to which this invention pertains. All references cited in this specification are to be taken as indicative of the level of knowledge in the matter. Nothing herein is to be construed as administration that the invention is not empowered to precede such disclosure by virtue of the above invention.

"Compounds of the invention" refers to the mono-, di- and trihydroxylated analogs of docosanoic acid that also have 5 or 6 double bonds in their carbon chain of 22 carbon atoms, and compounds encompassed by the generic formulas disclosed in herein, and include any specific compounds of these formulas whose structure is disclosed herein. The compounds of the invention can be identified by their chemical structure and/or chemical name. When the chemical structure and the chemical name do not correspond, the chemical structure will determine the identity of the compound. The compounds of the invention can contain one or more chiral centers and/or double bonds and, therefore, can exist as stereoisomers, such as double bond isomers (specifically geometric isomers), or enantiomers or diastereomers by presenting different S and R configurations at each chiral center. Accordingly, the chemical structures depicted herein encompass all possible enantiomers, epimers, diastereomers, and stereoisomers of the illustrated compounds, including the stereoisomerically pure form (e.g., geometrically pure, enantiomerically pure, or diastereoisomerically pure) and enantiomeric and stereoisomeric mixtures. Enantiomeric and stereoisomeric mixtures can be resolved into their enantiomeric components or component stereoisomers using separation techniques or asymmetric synthesis techniques well known to those skilled in the art. Compounds of the invention also include isotopically-labeled compounds in which one or more atoms have an atomic mass different from the atomic mass conventionally found in nature.

The compounds represented throughout the specification contain ethylenically unsaturated sites. When carbon-carbon double bonds exist, the configurational chemistry can be cis (Z) or trans (E) and the representations throughout the specification are not intended to be limiting. The representations are generally presented based on the configurational chemistry of related DHA, DPA, or EPA compounds, and although they are not limited in theory, they are believed to possess chemistry of similar configuration. The use of "-----" reflects this throughout the specification and claims, such that both cis and trans isomers are contemplated. In certain embodiments, the configuration of the ethylenic bond is known and is particularly described.

For clarity, the use of "-----" in parallel to a carbon-carbon bond in the Markush formulas of the present invention indicates the existence of a double bond that may have a cis (Z) configuration or a trans (E) configuration, regardless of the E or Z configuration that appears on a given double bond "-̅-̅-̅-̅-̅-̅" in a given Markush formula.

"Biological activity" and its contextual equivalents "activity" and "bioactivity" mean that a compound elicits a statistically valid effect in any biological test assay. Preferably, the threshold for defining an "active" compound will be reproducible and statistically valid effects of at least 25% deviation from the untreated control at concentrations of 1 microM or less.

"Biological test assay" means a specific experimental procedure. Non-limiting examples of biological test assays include: 1) ligand binding, direct or indirect, to a purified target, subcellular fraction, intact cell, or cell or tissue extract; 2) metabolic protection with enhanced half-life when exposed to a purified target, subcellular fraction, intact cell, cell or tissue extract, or administered to the intact organism by any route; 3) prevention, reversal or enhancement of cell- and tissue-based functional responses recognized by those skilled in the art to represent substitutes for anti-inflammatory action as for example, altered cytokine production and release, and 4) prevention, reversal or enhancement of symptoms and/or pathological processes in animal models of inflammation and inflammatory disease in relation to the release of cytokines.

"Detectable label" means any chemical or biological modality that can be used to trace, trace, locate, quantify, immobilize, purify, or identify compounds by appropriate detection means known in the art. Non-limiting examples of detectable labels include labels of fluorescence, phosphorescence, luminescence, radioactive or biospecific affinity capture.

"Electronegative group" is a chemical group that tends to acquire, rather than lose, electrons in its chemical interactions. Examples of electronegative groups include, but are not limited to, -NO2, ammonium salts, sulfonyl groups, carbonyl groups, halogens, esters, carboxylic acids, nitriles, etc.

"in situ" refers to and includes the terms "in vivo", "ex vivo" and "in vitro" as these terms are commonly recognized and understood by one of ordinary skill in the art. Furthermore, the phrase "in situ" is used herein in the broadest connotative and denotative context to identify an entity, cell, or tissue as found or in its place, regardless of its source or origin, its condition or state or its duration or longevity in that location or position.

"Pharmaceutically acceptable" means approved by a regulatory agency of the federal or state government or listed in the US Pharmacopeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) salts formed when a basic proton is present in the parent compound such as acid addition salts, formed with inorganic acids such as hydrochloric acid, hydrobromic acid, sulfuric acid, nitric acid, phosphoric acid, and the like; or those formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 2-naphthalenesulfonic acid 4-toluenesulfonic acid, camphorsulfonic acid, 4-methylbicyclo [2.2.2] -oct-2-en-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, and the like; or (2) salts formed when an acidic proton is present in the parent compound and is replaced by a metal ion, e.g., an alkali metal ion, an alkaline earth ion, or an aluminum ion; or it coordinates with an organic base such as ethanolamine, diethanolamine, triethanolamine, W-methylglucamine, triethylamine, propylamine, diazabicycloundecane and the like.

"Pharmaceutically acceptable carrier" refers to a diluent, adjuvant, excipient, or vehicle with which a compound of the invention is administered.

The phrase "pharmaceutically acceptable carrier" as used herein means a pharmaceutically acceptable material, composition, or carrier such as a liquid or solid filler, diluent, excipient, solvent, or encapsulating material involved in the carrying or transportation of a compound or compounds of the present invention in or to the subject in such a way that they can perform their intended function. Typically, such compounds are carried or transported from one organ, or portion of the body, to another organ or portion of the body. Each vehicle must be "acceptable" in the sense of being compatible with the other ingredients of the formulation and not detrimental to the patient. Some examples of materials that can serve as pharmaceutically acceptable carriers include: sugars such as lactose, glucose, and sucrose; starches such as corn starch and potato starch; cellulose and its derivatives, such as sodium carboxymethyl cellulose, ethyl cellulose and cellulose acetate; tragacanth powder; malt; jelly; talcum powder; excipients such as cocoa butter and suppository waxes; oils such as peanut oil, cottonseed oil, safflower oil, sesame oil, olive oil, corn oil, and soybean oil; glycols such as propylene glycol; polyols such as glycerin, sorbitol, mannitol, and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; phosphate buffers and other compatible non-toxic substances used in pharmaceutical formulations.

"Prodrug" refers to a derivative of a drug molecule that requires transformation in the body to release the active drug. Prodrugs are frequently (though not necessarily) pharmacologically inactive until they are converted to the parent drug. A hydroxyl-containing drug can be converted, for example, to a sulfonate, ester, or carbonate prodrug, which can be hydrolyzed in vivo to provide the hydroxyl compound. An amino-containing drug can be converted, for example, to a carbamate, amide, imine, phosphonyl, phosphoryl or sulfenyl prodrug, which can be hydrolyzed in vivo to provide the amino compound. A carboxylic acid drug can be converted to an ester (include silyl esters and thioesters), amide, or hydrazide prodrug, which can be hydrolyzed in vivo to provide the carboxylic acid compound. Prodrugs for drugs containing different functional groups other than those listed above are well known to those skilled in the art.

"Prorrest" refers to a form of protecting group that, when used to mask a functional group on a drug molecule, converts the drug into a prodrug. Typically, the pro-residue will bind to the drug through a linkage or linkages that are cleaved by enzymatic or non-enzymatic means in vivo.

"Protecting group" refers to a grouping of atoms that, when attached to a reactive functional group on a molecule, masks, reduces, or prevents the reactivity of the functional group. Examples of protecting groups can be found in Green et al., "Protective Groups in Organic Chemistry", (Wiley, 2nd ed. 1991) and Harrison et al., "Compendium of Synthetic Organic Methods" Vol. 1-8 (John Wiley and Sons, 1971-1996). Representative amino protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilylethanesulfonyl ("SES"), trityl, and trityl groups substituted, allyloxycarbonyl, 9-fluorenylmethyloxycarbonyl ("FMOC"), nitroveratryloxycarbonyl ("NVOC"), and the like. Representative hydroxyl protecting groups include, but are not limited to, those in which the hydroxyl group is acylated (e.g., methyl esters and ethyl, acetate groups or propionate or glycol esters) or alkylated ones such as trityl ethers, alkyl ethers, tetrahydropyranyl ethers, trialkylsilyl ethers (e.g., TMS or TIPPS groups) and allyl ethers.

"Subject" means living organisms susceptible to conditions or diseases caused or contributed by the presence of a pathogen in the respiratory system. Examples of subjects include humans, dogs, cats, cows, goats, and mice. The term subject is intended to further include transgenic species such as, for example, transgenic mice.

"Alkyl", by itself or as part of another substituent, refers to a monovalent, branched, linear or cyclic hydrocarbon radical, saturated or unsaturated, having the stated number of carbon atoms (i.e., C1-C6 means of 1 to 6 carbon atoms) and derived from the removal of a hydrogen atom from a single carbon atom from an original alkane, alkene or alkyne. Typical alkyl groups include, but are not limited to, methyl; ethyl such as ethanyl, ethenyl, ethynyl; propyls such as propan-1-yl, propan-2-yl, cyclopropan-1-yl, prop-1-en-1-yl, prop-1-en-2-yl, prop-2-en-1-yl , cycloprop-1-en-1-yl; cycloprop-2-en-1-yl, prop-1-y-1-yl, prop-2-y-1-yl, etc.; butyls such as butan-1-yl, butan-2-yl, 2-methyl-propan-1-yl, 2-methylpropan-2-yl, cyclobutan-1-yl, but-1-en-1-yl, but -1-en-2-yl, 2-methylprop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1 -in-1-yl, but-1-in-3-yl, but-3-y-1-yl, etc. and the like. When specific levels of saturation are intended, the nomenclature "alkanyl", "alkenyl" and/or "alkynyl" is used, as defined below. In preferred embodiments, the alkyl groups are (C1-C6) alkyl.

"Alkanyl", by itself or as part of another substituent, refers to a saturated cyclic, linear, or branched alkyl derived from the removal of a hydrogen atom from a single carbon atom from a parent alkane. Typical alkanyl groups include, but are not limited to, methanyl; ethanyl; propanyl such as propan-1-yl, propan-2-yl (isopropyl), cyclopropan-1-yl, etc.; butanyls such as butan-1-yl, butan-2-yl (sec-butyl), 2-methyl-propan-1-yl (isobutyl), 2-methylpropan-2-yl (tert-butyl), cyclobutan-1-ilo, etc., and the like. In preferred embodiments, the alkanyl groups are (C1-C6) alkanyl.

"Alkenyl" by itself or as part of another substituent refers to an unsaturated branched, straight-chain or cyclic alkyl having at least one carbon-carbon double bond derived by the removal of one hydrogen atom from a single carbon atom of a parent alkene. The group may be in either the cis or trans conformation about the double bond(s). Typical alkenyl groups include, but are not limited to, ethenyl; propenyls such as prop-1-en-1-yl, prop-1-en-2-yl, prop-2-en-1-yl, prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, etc.; and the like. In preferred embodiments, the alkenyl group is (C2-C6) alkenyl.

"Alkynyl" by itself or as part of another substituent refers to an unsaturated branched, straight-chain or cyclic alkyl having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, etc.; butynyls such as but-1-yn-1-yl, but-1-yn-3-yl, but-3-yn-1-yl, etc.; and the like. In preferred embodiments, the alkynyl group is (C2-C6) alkynyl.

"Alkyldiyl" by itself or as part of another substituent refers to a saturated or unsaturated, branched, straight-chain or cyclic divalent hydrocarbon group having the stated number of carbon atoms (i.e., C1-C6 means from one to six carbon atoms) derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkyldiyl groups include, but are not limited to, methandiyl; ethyldiyls such as ethan-1,1-diyl, ethan-1,2-diyl, ethen-1,1-diyl, ethen-1,2-diyl; propyldiyls such as propan-1,1-diyl, propan-1,2-diyl, propan-2,2-diyl, propan-1,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1,2-diyl, cycloprop-2-en-1,2-diyl, cycloprop-2-en-1,1-diyl, prop-1-yn-1,3-diyl, etc.; butyldiyls such as, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1-en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkanyldiyl, alkenyldiyl and/or alkynyldiyl is used. Where it is specifically intended that the two valencies are on the same carbon atom, the nomenclature "alkylidene" is used. In preferred embodiments, the alkyldiyl group is (C1-C6) alkyldiyl. Also preferred are saturated acyclic alkanyldiyl groups in which the radical centers are at the terminal carbons, e.g., methandiyl (methane); ethan-1,2-diyl (ethane); propan-1,3-diyl (propane); butan-1,4-diyl (butane); and the like (also referred to as alkylenes, defined infra).

"Alkdiyl" by itself or as part of another substituent refers to a saturated or unsaturated, branched, straight-chain or cyclic divalent hydrocarbon group having the stated number of carbon atoms (i.e., C1-C6 means from one to six carbon atoms) derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkdiyl groups include, but are not limited to methandiyl; ethyldiyls such as ethan-1,1-diyl, ethan-1,2-diyl, ethen-1,1-diyl, ethen-1,2-diyl; propyldiyls such as propan-1,1-diyl, propan-1 ,2-diyl, propan-2,2-diyl, propan-1 ,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1 ,2-diyl, cycloprop-2-en-1 ,2-diyl, cycloprop-2-en-1,1-diyl-, prop-1-yn-1 ,3-diyl, etc.; butyldiyls such as, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1-en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkandiyl, alkendiyl and/or alkyndiyl is used. In a preferred embodiment, the alkdiyl group is (C1-C6) alkdiyl. Also preferred are saturated acyclic alkanyldiyl groups in which the radical centers are at the terminal carbons, e.g., methandiyl (methane); ethan-1,2-diyl (ethane); propan-1,3-diyl (propane); butan-1,4-diyl (butane); and the like (also referred to as alkylenes, defined infra)

"Alkyleno" by itself or as part of another substituent refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkylene is indicated in square brackets. Typical alkylene groups include, but are not limited to, methane; ethylenes such as ethane, ethene, ethyne; propylenes such as propane, prop[1]ene, propa[1,2]diene, prop[1]yne, etc.; butylenes such as butane, but[1]ene, but[2]ene, buta[1,3]diene, but[1]yne, but[2]yne, buta[1,3]diyne, etc.; and the like. Where specific levels of saturation are intended, the nomenclature alkane, alkene and/or alkyne is used. In preferred embodiments, the alkyleno group is (C1-C6) or (C1-C3) alkylene. Also preferred are straight-chain saturated alkane groups, e.g., methane, ethane, propane, butane, and the like.

"Heteroalkyl," Heteroalkanyl," "Heteroalkenyl," "Heteroalkynyl," "Heteroalkyldiyl" and "Heteroalkyleno" by themselves or as part of another substituent refer to alkyl, alkanyl, alkenyl, alkynyl, alkyldiyl and alkyleno groups, respectively, in which one or more of the carbon atoms are each independently replaced with the same or different heteratoms or heteroatomic groups. Typical heteroatoms and/or heteroatomic groups which can replace the carbon atoms include, but are not limited to, -O-, -S-, -S-O-,-NR'-, -PH-, -S(O)-, -S(O)2-, -S(O) NR'-, -S(O)2NR'-, and the like, including combinations thereof, where each R' is independently hydrogen or (C1-C6) alkyl.

"Cycloalkyl" and "Heterocycloalkyl" by themselves or as part of another substituent refer to cyclic versions of "alkyl" and "heteroalkyl" groups, respectively. For heteroalkyl groups, a heteroatom can occupy the position that is attached to the remainder of the molecule. Typical cycloalkyl groups include, but are not limited to, cyclopropyl; cyclobutyls such as cyclobutanyl and cyclobutenyl; cyclopentyls such as cyclopentanyl and cyclopentenyl; cyclohexyls such as cyclohexanyl and cyclohexenyl; and the like. Typical heterocycloalkyl groups include, but are not limited to, tetrahydrofuranyl (e.g., tetrahydrofuran-2-yl, tetrahydrofuran-3-yl, etc.), piperidinyl (e.g., piperidin-1-yl, piperidin-2-yl, etc.), morpholinyl (e.g., morpholin-3-yl, morpholin-4-yl, etc.), piperazinyl (e.g., piperazin-1-yl, piperazin-2-yl, etc.), and the like.

"Acyclic Heteroatomic Bridge" refers to a divalent bridge in which the backbone atoms are exclusively heteroatoms and/or heteroatomic groups. Typical acyclic heteroatomic bridges include, but are not limited to, -O-, -S-, -S-O-, -NR'-, -PH-,-S(O)-, -S(O)2-, -S(O) NR'-, -S(O)2NR'-, and the like, including combinations thereof, where each R' is independently hydrogen or (C1-C6) alkyl.

"Parent Aromatic Ring System" refers to an unsaturated cyclic or polycyclic ring system having a conjugated π electron system. Specifically included within the definition of "parent aromatic ring system" are fused ring systems in which one or more of the rings are aromatic and one or more of the rings are saturated or unsaturated, such as, for example, fluorene, indane, indene, phenalene, tetrahydronaphthalene, etc. Typical parent aromatic ring systems include, but are not limited to, aceanthrylene, acenaphthylene, acephenanthrylene, anthracene, azulene, benzene, chrysene, coronene, fluoranthene, fluorene, hexacene, hexaphene, hexalene, indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, tetrahydronaphthalene, triphenylene, trinaphthalene, and the like, as well as the various hydro isomers thereof.

"Aryl" by itself or as part of another substituent refers to a monovalent aromatic hydrocarbon group having the stated number of carbon atoms (i.e., C5-C15 means from 5 to 15 carbon atoms) derived by the removal of one hydrogen atom from a single carbon atom of a parent aromatic ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene, and the like, as well as the various hydro isomers thereof. In preferred embodiments, the aryl group is (C5-C15) aryl, with (C5-C10) being even more preferred. Particularly preferred aryls are cyclopentadienyl, phenyl and naphthyl.

"Arylaryl" by itself or as part of another substituent refers to a monovalent hydrocarbon group derived by the removal of one hydrogen atom from a single carbon atom of a ring system in which two or more identical or non-identical parent aromatic ring systems are joined directly together by a single bond, where the number of such direct ring junctions is one less than the number of parent aromatic ring systems involved. Typical arylaryl groups include, but are not limited to, biphenyl, triphenyl, phenyl-naphthyl, binaphthyl, biphenyl-naphthyl, and the like. Where the number of carbon atoms in an arylaryl group are specified, the numbers refer to the carbon atoms comprising each parent aromatic ring. For example, (C5-C15) arylaryl is an arylaryl group in which each aromatic ring comprises from 5 to 15 carbons, e.g., biphenyl, triphenyl, binaphthyl, phenylnaphthyl, etc. Preferably, each parent aromatic ring system of an arylaryl group is independently a (C5-C15) aromatic, more preferably a (C5-C10) aromatic. Also preferred are arylaryl groups in which all of the parent aromatic ring systems are identical, e.g., biphenyl, triphenyl, binaphthyl, trinaphthyl, etc.

"Biaryl" by itself or as part of another substituent refers to an arylaryl group having two identical parent aromatic systems joined directly together by a single bond. Typical biaryl groups include, but are not limited to, biphenyl, binaphthyl, bianthracyl, and the like. Preferably, the aromatic ring systems are (C5-C15) aromatic rings, more preferably (C5-C10) aromatic rings. A particularly preferred biaryl group is biphenyl.

"Arylalkyl" by itself or as part of another substituent refers to an acyclic alkyl group in which one of the hydrogen atoms bonded to a carbon atom, typically a terminal or sp3 carbon atom, is replaced with an aryl group. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylakenyl and/or arylalkynyl is used. In preferred embodiments, the arylalkyl group is (C6-C21) arylalkyl, e.g., the alkanyl, alkenyl or alkynyl moiety of the arylalkyl group is (C1-C6) and the aryl moiety is (C5-C15). In particularly preferred embodiments the arylalkyl group is (C6-C13), e.g., the alkanyl, alkenyl or alkynyl moiety of the arylalkyl group is (C1-C3) and the aryl moiety is (C5-C10).

"Parent Heteroaromatic Ring System" refers to a parent aromatic ring system in which one or more carbon atoms are each independently replaced with the same or different heteroatoms or heteroatomic groups. Typical heteroatoms or heteroatomic groups to replace the carbon atoms include, but are not limited to, N, NH, P, O, S, S(O), S(O)2, Si, etc. Specifically included within the definition of "parent heteroaromatic ring systems" are fused ring systems in which one or more of the rings are aromatic and one or more of the rings are saturated or unsaturated, such as, for example, benzodioxan, benzofuran, chromane, chromene, indole, indoline, xanthene, etc. Also included in the definition of "parent heteroaromatic ring system" are those recognized rings that include common substituents, such as, for example, benzopyrone and 1-methyl-1,2,3,4-tetrazole. Typical parent heteroaromatic ring systems include, but are not limited to, acridine, benzimidazole, benzisoxazole, benzodioxan, benzodioxole, benzofuran, benzopyrone, benzothiadiazole, benzothiazole, benzotriazole, benzoxaxine, benzoxazole, benzoxazoline, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like.

"Heteroaryl" by itself or as part of another substituent refers to a monovalent heteroaromatic group having the stated number of ring atoms (e.g., "5-14 membered" means from 5 to 14 ring atoms) derived by the removal of one hydrogen atom from a single atom of a parent heteroaromatic ring system. Typical heteroaryl groups include, but are not limited to, groups derived from acridine, benzimidazole, benzisoxazole, benzodioxan, benzodiaxole, benzofuran, benzopyrone, benzothiadiazole, benzothiazole, benzotriazole, benzoxazine, benzoxazole, benzoxazoline, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like, as well as the various hydro isomers thereof. In preferred embodiments, the heteroaryl group is a 5-14 membered heteroaryl, with 5-10 membered heteroaryl being particularly preferred.

"Heteroaryl-Heteroaryl" by itself or as part of another substituent refers to a monovalent heteroaromatic group derived by the removal of one hydrogen atom from a single atom of a ring system in which two or more identical or non-identical parent heteroaromatic ring systems are joined directly together by a single bond, where the number of such direct ring junctions is one less than the number of parent heteroaromatic ring systems involved. Typical heteroaryl-heteroaryl groups include, but are not limited to, bipyridyl, tripyridyl, pyridylpurinyl, bipurinyl, etc. Where the number of atoms are specified, the numbers refer to the number of atoms comprising each parent heteroaromatic ring systems. For example, 5-15 membered heteroaryl-heteroaryl is a heteroaryl-heteroaryl group in which each parent heteroaromatic ring system comprises from 5 to 15 atoms, e.g., bipyridyl, tripuridyl, etc. Preferably, each parent heteroaromatic ring system is independently a 5-15 membered heteroaromatic, more preferably a 5-10 membered heteroaromatic. Also preferred are heteroaryl-heteroaryl groups in which all of the parent heteroaromatic ring systems are identical.

"Biheteroaryl" by itself or as part of another substituent refers to a heteroaryl-heteroaryl group having two identical parent heteroaromatic ring systems joined directly together by a single bond. Typical biheteroaryl groups include, but are not limited to, bipyridyl, bipurinyl, biquinolinyl, and the like. Preferably, the heteroaromatic ring systems are 5-15 membered heteroaromatic rings, more preferably 5-10 membered heteroaromatic rings. "Heteroarylalkyl" by itself or as part of another substituent refers to an acyclic alkyl group in which one of the hydrogen atoms bonded to a carbon atom, typically a terminal or sp3 carbon atom, is replaced with a heteroaryl group. Where specific alkyl moieties are intended, the nomenclature heteroarylalkanyl, heteroarylakenyl and/or heteroarylalkynyl is used. In preferred embodiments, the heteroarylalkyl group is a 6-21 membered heteroarylalkyl, e.g., the alkanyl, alkenyl or alkynyl moiety of the heteroarylalkyl is (C1-C6) alkyl and the heteroaryl moiety is a 5-15-membered heteroaryl. In particularly preferred embodiments, the heteroarylalkyl is a 6-13 membered heteroarylalkyl, e.g., the alkanyl, alkenyl or alkynyl moiety is (C1-C3) alkyl and the heteroaryl moiety is a 5-10 membered heteroaryl.

"Halogen" or "Halo" by themselves or as part of another substituent, unless otherwise stated, refer to fluoro, chloro, bromo and iodo.

"Haloalkyl" by itself or as part of another substituent refers to an alkyl group in which one or more of the hydrogen atoms is replaced with a halogen. Thus, the term "haloalkyl" is meant to include monohaloalkyls, dihaloalkyls, trihaloalkyls, etc. up to perhaloalkyls. For example, the expression "(C1-C2) haloalkyl" includes fluoromethyl, difluoromethyl, trifluoromethyl, 1-fluoroethyl, 1,1-difluoroethyl, 1,2-difluoroethyl, 1,1,1-trifluoroethyl, perfluoroethyl, etc.

The above-defined groups may include prefixes and/or suffixes that are commonly used in the art to create additional well-recognized substituent groups. As examples, "alkyloxy" or "alkoxy" refers to a group of the formula -OR", "alkylamine" refers to a group of the formula -NHR" and "dialkylamine" refers to a group of the formula -NR"R", where each R" is independently an alkyl. As another example, "haloalkoxy" or "haloalkyloxy" refers to a group of the formula -OR‴, where R‴ is a haloalkyl.

The present invention is also directed to methods or procedures for treating or preventing infections caused by a pathogen in the respiratory system as for example but not limited to: pneumonia, bacterial pneumonia, viral pneumonia, atypical pneumonia, graft versus host disease (GVHD), coronavirus disease 2019 (COVID-19), sepsis, Ebola, bird flu, smallpox, common flu, pancreatitis, a viral infection, or infections associated with acute respiratory distress syndrome (ARDS), acute respiratory syndrome severe (SARS), systemic inflammatory response syndrome (SIRS), or Severe fever with thrombocytopenia syndrome (SFTS), by administering to an individual in need, of an effective amount of any one of the compounds, compositions or pharmaceutical compositions described herein document.

In the present invention, the expression "respiratory system" or respiratory apparatus refers to the entire set of organs and structures possessed by living beings whose purpose is the exchange of gases with the environment.

The pharmaceutical compositions of the invention include a "therapeutically effective amount" or a "prophylactically effective amount" of one or more of the unsaturated docosanoic acid analog of the invention. A "therapeutically effective amount" refers to an amount effective, at dosages and for periods of time necessary, to achieve the desired therapeutic result, e.g., a diminishment or prevention of effects associated with various disease states or conditions. A therapeutically effective amount of unsaturated docosanoic acid analog of the invention may vary according to factors such as the disease state, age, sex, and weight of the individual, and the ability of the therapeutic compound to elicit a desired response in the individual. A therapeutically effective amount is also one in which any toxic or detrimental effects of the therapeutic agent are outweighed by the therapeutically beneficial effects.

A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount. Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the unsaturated docosanoic acid analog and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of a unsaturated docosanoic acid analog of the invention is 0.01-20 mg/kg, more preferably 0.1-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

When the compounds of the present invention are administered as pharmaceuticals, to humans and mammals, they can be given per se or as a pharmaceutical composition containing, for example, 0.1 to 99.5% (more preferably, 0.5 to 90%) of active ingredient, i.e., at least one unsaturated docosanoic acid analog, in combination with a pharmaceutically acceptable carrier.

In certain embodiments, the compounds of the present invention may contain one or more acidic functional groups and, thus, are capable of forming pharmaceutically acceptable salts with pharmaceutically acceptable bases. The term "pharmaceutically acceptable salts, esters, amides, and prodrugs" as used herein refers to those carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use of the compounds of the invention.

The term "salts" refers to the relatively nontoxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. These may include cations based on the alkali and alkaline earth metals, such as sodium, lithium, potassium, calcium, magnesium and the like, as well as nontoxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S. M., et al., "Pharmaceutical Salts" J. Pharm. Sci., 1977; 66:1 19 which is incorporated herein by reference).

The term "pharmaceutically acceptable esters" refers to the relatively non-toxic, esterified products of the compounds of the present invention. These esters can be prepared in situ during the final isolation and purification of the compounds, or by separately reacting the purified compound in its free acid form or hydroxyl with a suitable esterifying agent. Carboxylic acids can be converted into esters via treatment with an alcohol in the presence of a catalyst. The term is further intended to include lower hydrocarbon groups capable of being solvated under physiological conditions, e.g., alkyl esters, methyl, ethyl and propyl esters.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Formulations of the present invention include those suitable for intravenous, oral, nasal, topical, transdermal, buccal, sublingual, rectal, vaginal and/or parenteral administration. The formulations may conveniently be presented in unit dosage form and may be prepared by any methods well known in the art of pharmacy. The amount of active ingredient which can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent. The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. They may also include anionic modified celluloses such as: microcrystalline cellulose, sodium carboxymethylcellulose, ethylhydroxyethylcellulose, hydroxypropylmethylcellulose (HPMC), hydroxyethylmethylcellulose, hydroxypropylcellulose (HPC), hydroxyethylcellulose, ethylmethylcellulose, ethylcellulose, and/or methylcellulose, and an hydrophilic emulsifier.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Ophthalmic formulations, eye ointments, powders, solutions and the like, are also contemplated as being within the scope of this invention. Such solutions are useful for the treatment of conjunctivitis.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Intravenous injection administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systematically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of ordinary skill in the art. Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, intravenous and subcutaneous doses of the compounds of this invention for a patient, when used for the indicated analgesic effects, will range from about 0.0001 to about 100 mg per kilogram of body weight per day, more preferably from about 0.01 to about 50 mg per kg per day, and still more preferably from about 0.1 to about 40 mg per kg per day. For example, between about 0.01 microgram and 20 micrograms, between about 20 micrograms and 100 micrograms and between about 10 micrograms and 200 micrograms of the compounds of the invention are administered per 20 grams of subject weight.

If desired, the effective daily dose of the active compound may be administered as two, three, four, five, six or more sub-doses administered separately at appropriate intervals throughout the day, optionally, in unit dosage forms.

The invention features an article of manufacture that contains packaging material and unsaturated docosanoic acid analog formulation contained within the packaging material. This formulation contains an at least one unsaturated docosanoic acid analog and the packaging material contains a label or package insert indicating that the formulation can be administered to the subject to treat one or more conditions as described herein, in an amount, at a frequency, and for a duration effective to treat or prevent such condition(s). Such conditions are mentioned throughout the specification and are incorporated herein by reference. Suitable unsaturated docosanoic acid analogs are described herein.

The present invention provides novel uses of compounds and compositions relating to unsaturated analogues of mono-, di- and trihydroxyl of docosanoic acid characterized mainly by having a hydroxyl group at C-14 of the carbon chain.

According to the present description, the use of a compound of the invention or of a pharmaceutical composition for the manufacture of a medicine or alternatively its use as a medicine, for the treatment of a disorder, disorder or disease described above, can obviously be understood as a method of treating such disorder, disorder or disease, which comprises administering to a subject a therapeutically effective amount of said compound or pharmaceutical composition of the invention. In other words, the present invention also relates to a method of treating a disorder, disorder or disease comprising administering to a subject the compound of the invention in a therapeutically effective amount, or a pharmaceutical composition of the invention comprising the compound of the invention in a therapeutically effective amount.

### DESCRIPTION OF THE DRAWINGS

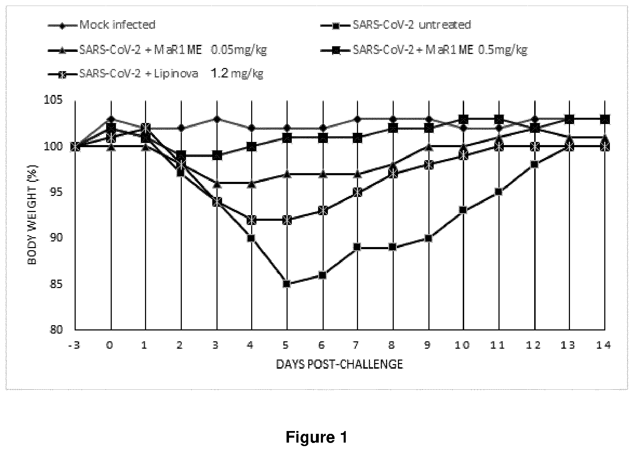

**Fig. 1****.** Shows the weight variation of the group of animals infected with SARS-CoV-2, treated or not with a compound of formula (I), the methyl ester of 7R,14S-maresin 1 (MaR1 ME) and Lipinova^{®} (a composition comprising the compound of formula (IV), 14S-hydroxy-4Z,7Z,10Z,12E,16Z,19Z-docosahexaenoic acid, in the triglyceride form, or 14-HDHA), compared to the untreated group not inoculated (Control). Thus, it can be observed that body weight loss decreases significantly in animals exposed to SARS-CoV-2 treated with MaR1 ME and Lipinova^{®}. In the case of groups treated with MaR1 ME, the improvement in weight loss is dose dependent, the highest dose of MaR1 ME leads to a greatly reduced body weight loss, of less than 5%.
**Fig. 2****.** Shows the TNF-α cytokine profile obtained by the qRT-PCR assay in the lung tissues of SARS-CoV-2 infected hamsters treated and untreated at 4 dpi, 7 dpi and 14 dpi (n = 6 per day). Treatments began on the second day after infection.
**Fig. 3****.** Shows the effect produced by treatment with MaR1 ME and Lipinova^{®} of infected animals, finding that the viral load in the lungs is significantly reduced after a few days of treatment.

### EXAMPLES

### Example 1. Determination of the content of SPMs in plasma

Obtaining plasma samples: For the present assay, blood was collected from (5) COVID-19 patients and (5) healthy subjects. For this, butterfly needles of 21 caliber were used to draw blood into a 10 ml syringe containing heparin (10 units / ml). The collected sample was then transferred to a 15 ml polypropylene tube and centrifuged for 20 minutes at 120G. The supernatant (plasma) was then collected with a Pasteur pipette and placed in another 15 ml polypropylene tube. The tube with the plasma was stored at -80 ° C. Processing prior to LC-MS / MS analysis: the plasma sample was thawed at room temperature, centrifuged at 1000G for 30 minutes before solid phase extraction. Internal standards (500 pg each) d4-PGE2, d5-LXA4, d4-LTB4, d8-5S-HETE, and d5-RvD2, representing each region in chromatographic analysis, were added to the sample in 4V of methanol. To induce protein precipitation, the samples were kept at -20 ° C for 45 min. The supernatant was taken and a solid phase extraction was carried out. The eicosanoids and SPMs bound to the matrix were dried and suspended in a methanol / H2O (1:1) mixture to be injected into a system of LC-MS / MS mass spectrometer of hybrid triple quadrupole SCIEX QTRAP 5500.

Concentration of SPMs (pg/ml) in human plasma samples from COVID-19 patients and healthy subjects (nd = not detected).

**Table 1. Bioactive metabolome related to docosahexaenoic acid (DHA)**

| | **RvD1** | **RvD2** | **RvD3** | **RvD4** | **RvD5** | **PDX** | **MaR1** |
|---|---|---|---|---|---|---|---|
| **Healthy (n=5)** | 3.1±0.5 | 3.1±0.5 | 0.4±0.4 | 1.2±0.4 | 1.8±0.4 | 1.2±0.7 | 0.6±0.2 |
| **COVID-19 (n=5)** | 2.4±0.6 | 2.1±0.6 | 0.3±0.3 | 0.3±0.3 | 1.3±0.4 | 0.5±0.4 | nd |

**Table 2. Bioactive metabolome related to eicosapentaenoic acid (EPA)**

| | **RvE1** | **RvE2** | **RvE3** |
|---|---|---|---|
| **Healthy (n=5)** | 4.0±1.0 | 2.4±1.1 | 1.3±0.6 |
| **COVID-19 (n=5)** | 2.2±1.0 | 2.0±1.0 | 1.6±0.7 |

**Table 3. Bioactive metabolome related to arachidonic acid (AA)**

| | **LXA₄** | **LXB₄** | **LXB₂** | **PGD₂** | **PGE₂** | **PGF**_{**2**α} | **LTB₄** |
|---|---|---|---|---|---|---|---|
| **Healthy (n=5)** | nd | nd | 3.1±1.3 | 5.5±2.2 | 3.6±1.1 | nd | 1.4±0.7 |
| **COVID-19 (n=5)** | 5.2±0.6 | 2.7±0.2 | 7.9±2.3 | 24±4.4 | 12.4±2.5 | 2.7±0.3 | 3.7±0.6 |

The analysis of the bioactive metabolome of Covid-19 patients shows a number of differences from the metabolome of healthy people. In the case of the compounds of the present invention, represented by the compound of formula (I), 7R,14S-dihydroxy-4Z, 8E,10E,12Z,16Z,19Z-docosahexaenoic acid (MaR1), it is surprisingly observed that unlike healthy individuals, this lipid mediator is not present in blood (plasma) or, if it is found, it does so at undetected concentrations, in Covid-19 patients.

In addition, marked decreases are observed in the concentration of RvE1 (-45%), RvD2 (-32%), RvD4 (-75%), and PDX (-58%) in plasma. These highly differentiated values between healthy people and Covid-19 patients indicate that these pro-resolution lipid mediators are relevant in this pathology. A decrease in the values of these lipid mediators in a subject are indicative of suffering from a disease caused by a pathogen in the respiratory system, while a patient suffering from one of the diseases described in the present invention whose levels of RvE1, RvD2, RvD4, PDX, and MaR1 are increased over time will be indicative of patient improvement.

On the other hand, a substantial increase is observed in two pro-inflammatory lipid mediators, PGD2 (+ 436%), and LTB4 (+ 264%). Therefore, these lipid mediators are relevant in this pathology. Its decrease in time will indicate that the patient tends towards an optimal or healthy state, while its increase is indicative of a worsening of the patient.

### Example 2.

### Virus

SARS-CoV-2 virus was isolated from the nasopharyngeal aspirate specimen of a laboratory-confirmed COVID-19 patient. The plaque purified viral isolate was amplified by one additional passage in VeroE6 cells to make working stocks of the virus as described previously.( Chan JF, Yip CC, To KK, et al. Improved molecular diagnosis of COVID-19 by the novel, highly sensitive and specific COVID-19-RdRp/Hel real-time reverse transcription-polymerase chain reaction assay validated in vitro and with clinical specimens. J Clin Microbiol., 2020; March 4).

### Animal experiments

Male LVG Golden Syrian Hamster (Body Weight of 100-150g, from Charles River, strain code 049) were kept in biosafety Level-2 housing and given access to standard pellet feed and water ad libitum for 6-10 days prior to the virus challenge, which was made in biosafety Level-3 animal facility. Phosphate-buffered saline (PBS) was used to dilute virus stocks to the desired concentration, and inocula were back-titrated to verify the dose given. Dulbecco's Modified Eagle Medium (DMEM) containing 105 plaque-forming units in 100µl of SARS-CoV-2 was intranasally inoculated under intraperitoneal ketamine (200mg/kg) and xylazine (10mg/kg) anesthesia. Mock-infected animals were challenged with 100ul of PBS. During all the experiment, animals were monitored twice daily for clinical signs of disease. Their body weight and survival were monitored for 14 days post-inoculation.

Seventy-six (76) hamsters were inoculated with SARS-CoV-2 virus titrated solution on Day 0, twelve (12) animals were mock inoculated the same day. Body weight and clinical signs were evaluated daily and twice daily respectively, starting three days before inoculation (Day -3). On day 2 post inoculation, four (4) inoculated animals and three (3) mock inoculated animals were sacrificed were euthanized by intra-peritoneal injection (i.p.) of pentobarbital at 200mg/kg and appropriate tissues (nasal turbinate, trachea, lungs, selected portion of GI tract) were collected for viral load and histopathology. Blood profiles of selected cytokine/chemokine (TNF-α, IL-6, IL-1, IL-8, and MCP-1) and compounds of the present invention (Methyl 7R,14S-dihydroxy-4Z,8E,10E,12Z,16Z,19Z-docosahexaenoate or MaR1 ME which is a compound of formula (I), and Lipinova^{®}, which is a composition comprising 14S-hydroxy-4Z,7Z,10Z,12E,16Z,19Z-docosahexaenoic acid or 14-HDHA, which is a compound of formula (IV), in the triglyceride form) were also measured. The remaining inoculated animals (72) animals were separated into four (4) cohorts of eighty (18) animals. Two cohorts were treated twice daily (i.p.) with MaR1 ME (at dosages of 0.05 and 0.5 mg/kg), one cohort was treated twice daily i.p. with vehicle, and the remaining group was treated twice daily (by gavage) with Lipinova^{®}.

On day 4, six (6) animals from each inoculated cohorts (treated and non-treated) and three (3) mock inoculated animals were sacrificed, appropriate tissues were collected for viral load and histopathology, blood cytokine/chemokine profiles were measured. On day 7, six (6) animals from each inoculated cohorts (treated and non-treated) and three (3) mock inoculated animals were sacrificed, appropriate tissues were collected for viral load and histopathology, blood cytokine/chemokine and SPM profiles were measured.

On day 14, six (6) animals from each inoculated cohorts (treated and non-treated) and three (3) mock inoculated animals were sacrificed, appropriate tissues were collected for viral load and histopathology, blood cytokine/chemokine and SPM profiles were measured. Blood and major organ tissues at necropsy were separated into two parts, one immediately fixed in 10% PBS-buffered formalin, the other immediately frozen at - 80°C until use.

**Table 4. General description of the animal test.**

| **Group Code** | **Nº** | **Group description** | **Inoculation** | **Nº-Day 2** | **Inicio Tratamiento** | **Treatment** | **Nº - Day 4** | **Nº - Day 7** | **Nº - Day 14** |
|---|---|---|---|---|---|---|---|---|---|
| 1 | 12 | Inoculados simulados control | PBS | 3* | 2 day post inoculation | Vehicle | 3 | 3 | 3 |
| 2 | 19 | Inoculados control | COVID-19 | 1* | | Vehicle | 6 | 6 | 6 |
| 3 | 19 | Inoculados tratados | | 1* | | Marl ME 0.05 mg/kg | 6 | 6 | 6 |
| 4 | 19 | Inoculados tratados | | 1* | | Marl ME 0.5 mg/kg | 6 | 6 | 6 |
| 5 | 19 | Inoculados tratados | | 1* | | Lipinova^{®} (1.20 mg/kg) | 6 | 6 | 6 |

| | | | | | | | | | |
|---|---|---|---|---|---|---|---|---|---|
| * Basal injury | | | | | | | | | |

### Variation in body weight

The variation of the body weight of the test animals of the group of animals infected with SARS-CoV-2, treated or not with a compound of formula (I), MaR1 ME and Lipinova^{®} (composition comprising 14- HDHA, a compound of formula (IV), in the triglyceride form), and the results were compared with those of the uninoculated and untreated group (Control). Thus, animals inoculated with SARS-CoV-2 but not treated exhibited a progressive loss of body weight of up to ∼ 15% between days 1 and 5 post injection (dpi), and then gradually regained their weight until 14 dpi. These animals developed lethargy, curly skins, hunched posture, and rapid breathing from 2 dpi, and began to recover at 7 dpi. While in animals exposed to SARS-CoV-2 and treated with MaR1 ME and Lipinova^{®}, it can be observed that the loss of body weight decreases significantly. In the case of the MaR1 ME treated groups, the improvement in weight loss is dose dependent, the highest dose of MaR1 ME leading to the lowest body weight loss, which was less than 5%. None of the SARS-CoV-2 infected or mock animals died.

### Histopathology and immunohistochemistry.

Tissue (nasal turbinate, trachea, lungs, selected portion of gastrointestinal tract) were fixed in 4% paraformaldehyde and were processed for paraffin embedding. The 4 µm sections were stained with hematoxylin and eosin for histopathological examinations. For immunohistochemistry, SARS-CoV-2 N protein was detected using monoclonal antibody (4D11) (Nicholls, M. *et al.* "Time course and cellular localization of SARS-CoV nucleoprotein and RNA in lungs from fatal cases of SARS". PLoS Med 3, e27, (2006)).

### Cytokine/chemokine profile

Chemokine/cytokine profiling was performed on the lung tissues and blood of the virus-challenged and mock-infected animals by qRT-PCR (Espitia CM, Zhao W, Saldarriaga O, et al. "Duplex real-time reverse transcriptase PCR to determine cytokine mRNA expression in a hamster model of New World cutaneous leishmaniasis". BMC Immunol 2010; 11: 31).

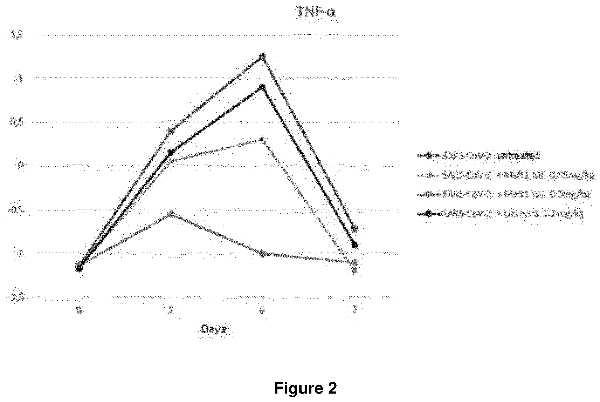

Both treatment with MaR1 ME and Lipinova^{®} have both a significant impact on the chemokine and cytokine profile in infected animals. Figure 2 specifically illustrates the increased profile of the cytokine TNF-α in the lungs of the group of animals infected with SARS-CoV-2 where the animals that have been treated in parallel with MaR1 ME and Lipinova^{®} present levels of TNF -α significantly lower than untreated animals.

Viral load determination by quantitative real-time RT-PCR.

RNA was extracted from 140 µL tissue homogenate using QIAamp viral RNA mini kit (Qiagen) and eluted with 60 µL of water. The N gene of SARS-CoV-2 virus was detected and quantified using TaqMan^{™} Fast Virus 1-Step Master Mix as described (Chu, K. W. et al. "Molecular Diagnosis of a Novel Coronavirus (2019-nCoV) Causing an Outbreak of Pneumonia". Clin Chem, doi:10.1093/clinchem/hvaa029 (2020).

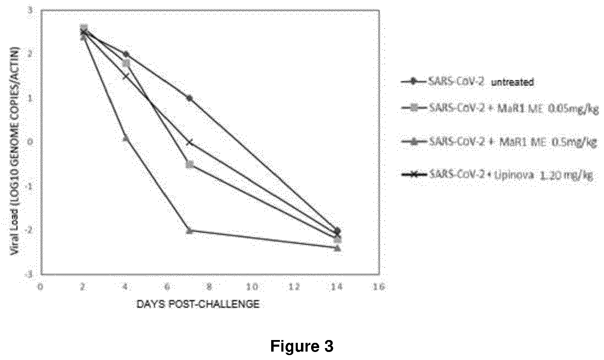

MaR1 ME and Lipinova^{®} treatments have a significant impact on viral load in tissues. Figure 3 shows that in infected animals treated with MaR1 ME and Lipinova^{®}, the viral load in the lungs is significantly reduced after a few days of treatment.

## Claims

1. An unsaturated analog of docosanoic acid of formula (I): 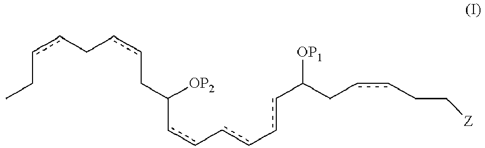
wherein each of P₁ and P₂ individually is a protecting group or a hydrogen atom;
wherein is -̅ -̅ -̅ -̅ -̅ -̅ a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, —NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c}or-[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for use in the treatment of an infection caused by a pathogen in the respiratory system, wherein are expressly excluded:
7R,14S-Dihydroxy-4Z,8E,10E,12Z,16Z,19Z-docosahexaenoic acid;
7S,14S-Dihydroxy-4Z,8E,10Z,12E,16Z,19Z-docosahexaenoic; and
7S,14S-Dihydroxy-4Z,8E,10E,12Z,16Z,19Z-docosahexaenoic acid,
when the pathogen is not Covid-19.

2. An unsaturated docosanoic acid analog of formula (I) according to claim 1, wherein P₁ and P₂ are both hydrogen atoms.

3. An unsaturated analog of docosanoic acid of formula (I) according to claim 1, wherein C-8 and C-10 double bonds are in the E configuration.

4. An unsaturated analog of docosanoic acid of formula (I) according to claim 1, wherein C-14 hydroxyl has S configuration.

5. An unsaturated analog of docosanoic acid of formula (II): 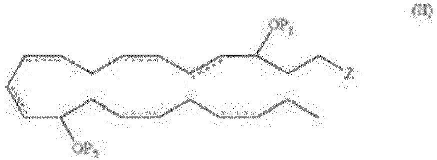
wherein each individually P₁ and P₂ is a protecting group or a hydrogen atom;
wherein -̅ -̅ -̅ -̅ -̅ -̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, —NO, —NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for use in the treatment of an infection caused by a pathogen in the respiratory system.

6. An unsaturated analog of docosanoic acid of formula (II) according to claim 5, wherein P₁ and P₂ are both hydrogen atoms.

7. An unsaturated analog of docosanoic acid of formula (II) according to claim 6, wherein double bonds at C-5 and C-12 positions are each in the E configuration.

8. An unsaturated analog of docosanoic acid of formula (II) according to claim 7, wherein C-14 hydroxyl has S configuration.

9. An unsaturated analog of docosanoic acid of formula (III): 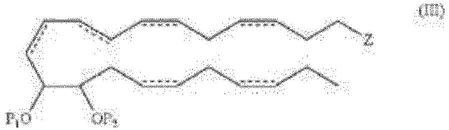
wherein each individually P₁ and P₂ is a protecting group or a hydrogen atom;
wherein -̅ -̅ -̅ -̅ -̅ -̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for use in the treatment of an infection caused by a pathogen in the respiratory system, where 13R,14S-Dihydroxy-4Z,7Z,9E,11E,16Z,19Z-docosahexaenoic acid is expressly excluded, when the pathogen is not Covid-19.

10. An unsaturated analog of docosanoic acid of formula (III) according to claim 9, wherein P₁ and P₂ are both hydrogen atoms.

11. An unsaturated analog of docosanoic acid of formula (III) according to claim 10, where Z is -C (O) OR^{d}, where R^{d} for Z is other than hydrogen.

12. An unsaturated analog of docosanoic acid of formula (III) according to claim 11, wherein C-14 hydroxyl has S configuration.

13. An unsaturated analog of docosanoic acid of formula (IV): 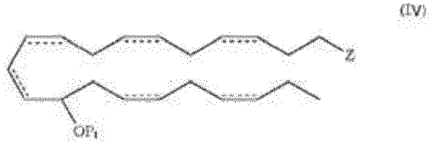
wherein P₁ is a protecting group or a hydrogen atom;
wherein - is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for the use in the treatment of an infection caused by a pathogen in the respiratory system, where are expressly excluded:
14-Hydroxy-4Z,7Z,10Z,12Z,16Z,19Z-docosahexaenoic acid;
14-Hydroxy-4Z,7Z,10Z,12E,16Z,19Z-docosahexaenoic acid; and
Methyl 14-hydroxy-4Z,7Z,10Z,12E,16Z,19Z-docosahexaenoate,
when the pathogen is not Covid-19.

14. An unsaturated analog of docosanoic acid of formula (IV) according to claim 13, wherein P₁ is a hydrogen atom.

15. An unsaturated analog of docosanoic acid of formula (IV) according to claim 14, where Z is -C(O)OR^{d}, where R^{d} for Z is hydrogen.

16. An unsaturated analog of docosanoic acid of formula (IV) according to claim 15, wherein the double bond at C-12 position is in the E configuration.

17. An unsaturated analog of docosanoic acid of formula (IV) according to claim 16, wherein C-14 hydroxyl has S configuration.

18. An unsaturated analog of docosanoic acid of formula (V): 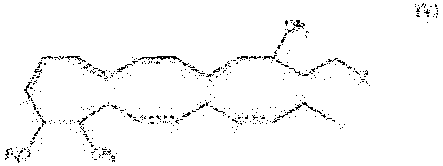
wherein each of P₁, P₂ and P₃ individually is a protecting group or a hydrogen atom;
wherein -̅ -̅ -̅ -̅ -̅ -̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, —NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c}or -[NR^{a}C(NR^{a})]ₙNR^{c}R^{c}; each n, independently is an integer from 0 to 3;
each R^{d}, independently is a protecting group or R^{a};
or a pharmaceutically acceptable salt, tautomer, and/or solvate thereof;
for the use in the treatment of an infection caused by a pathogen in the respiratory.

19. An unsaturated analog of docosanoic acid of formula (V) according to claim 18, wherein P₁, P₂ and P₃ are all hydrogen atoms.

20. An unsaturated analog of docosanoic acid of formula (V) according to claim 19, wherein Z is -C(O)OR^{d}.

21. An unsaturated analog of docosanoic acid of formula (V) according to claim 20, wherein C-14 hydroxyl has S configuration.

22. An unsaturated analog of docosanoic acid of formula (VI): 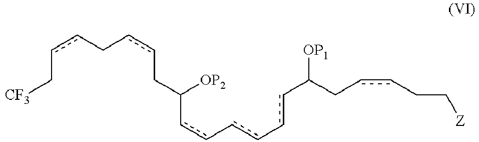
wherein each of P₁ and P₂, individually, is a protecting group or a hydrogen atom;
wherein -̅ -̅ -̅ -̅ -̅ -̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, -S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c}or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for the use in the treatment of an infection caused by a pathogen in the respiratory.

23. An unsaturated analog of docosanoic acid of formula (VI) according to claim 22, wherein P₁ and P₂ are both hydrogen atoms.

24. An unsaturated analog of docosanoic acid of formula (VI) according to claim 23, wherein Z is -C(O)OR^{d}.

25. An unsaturated analog of docosanoic acid of formula (VI) according to claim 24, wherein the C-14 hydroxyl has S configuration.

26. An unsaturated analog of docosanoic acid of formula (VII): 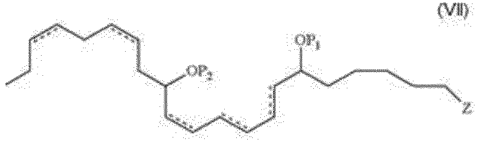
wherein each of P₁ and P₂, individually, is a protecting group or a hydrogen atom;
wherein -̅ -̅ -̅ -̅ -̅ -̅ is a double bond;
wherein Z is -C(O)OR^{d}, -C(O)NR^{c}R^{c}, -C(O)H, -C(NH)NR^{c}R^{c}, -C(S)H, -C(S)OR^{d}, -C(S)NR^{c}R^{c}, or -CN;
wherein each R^{a}, is independently selected from hydrogen, (C1-C6) alkyl, (C3-C8) cycloalkyl, cyclohexyl, (C4-C11) cycloalkylalkyl, (C5-C10) aryl, phenyl, (C6-C16) arylalkyl, benzyl, 2-6 membered heteroalkyl, 3-8 membered cycloheteroalkyl, morpholinyl, piperazinyl, homopiperazinyl, piperidinyl, 4-11 membered cycloheteroalkylalkyl, 5-10 membered heteroaryl or 6-16 membered heteroarylalkyl; wherein each R^{c}, is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
wherein each R^{b} is independently selected from =O, -OR^{d}, (C1-C3) haloalkyloxy, - OCF₃, =S, -SR^{d}, =NR^{d}, =NOR^{d}, -NR^{c}R^{c}, halogen, -CF₃, -CN, -NC, -OCN,-SCN, -NO, -NO₂, =N₂, -N₃, -S(O)R^{d}, -S(O)₂R^{d}, -S(O)₂OR^{d}, —S(O)NR^{c}R^{c},-S(O)₂NR^{c}R^{c}, -OS(O)R^{d}, -OS(O)₂R^{d}, -OS(O)₂OR^{d}, -OS(O)₂NR^{c}R^{c}, -C(O)R^{d},-C(O)OR^{d}, -C(O)NR^{c}R^{c}, -C(NH)NR^{c}R^{c}, -C(NR^{a})NR^{c}R^{c}, -C(NOH)R^{a},-C(NOH)NR^{c}R^{c}, -OC(O)R^{d}, -OC(O)OR^{d}, -OC(O)NR^{c}R^{c}, -OC(NH)NR^{c}R^{c},-OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙR^{d}, - [NHC(O)]ₙNR^{c}R^{c}, -[NR^{a}C(O)O]ₙNR^{c}R^{c}, -[NHC(NH)]ₙNR^{c}R^{c} or -[NR^{a}C(NR^{a})]ₙNR^{c}R^{c};
each n, independently is an integer from 0 to 3;
each R^{d}, independently is a protecting group or R^{a};
or a pharmaceutically acceptable salt, tautomer, and/or solvate thereof;
for the use in the treatment of an infection caused by a pathogen in the respiratory, where 7,14-Dihydroxy-8E,10E,12Z,16Z,19Z-eicosapentaenoic acid is expressly excluded, when the pathogen is not Covid-19.

27. An unsaturated analog of docosanoic acid of formula (VII) according to claim 26, wherein P₁ and P₂ are both hydrogen atoms.

28. An unsaturated analog of docosanoic acid of formula (VII) according to claim 27, wherein Z is -C(O)OR^{d}.

29. An unsaturated analog of docosanoic acid of formula (VII) according to claim 28, wherein the C-14 hydroxyl has S configuration.

30. A pharmaceutical composition comprising at least one compound according to any one of claims 1 to 29, and a pharmaceutically acceptable carrier, for use in the treatment of an infection caused by a pathogen in the respiratory system.

31. A pharmaceutical composition comprising at least one compound according to any one of claims 1 to 29, and the compounds 17-hydroxy-4Z,7Z,10Z,13Z,15E,19Z-docosahexaenoic acid ("17-HDHA"), and 18-hydroxy-5Z,8Z,11Z,14Z,16E-eicosapentaenoic acid ("18-HEPE") for use in the treatment of an infection caused by a pathogen in the respiratory system.

32. A pharmaceutical composition according to claim 31, wherein the compound according to any one of claims 1 to 29, and the compounds 17-HDHA, and 18-HEPE are in the free form, as esters, ethyl esters, triglycerides, diglycerides, monoglycerides, sphingolipids, phospholipids, or mixtures thereof.

33. A pharmaceutical composition according to any one of claims 30 to 32, wherein the infection caused by a pathogen in the respiratory system is selected from: pneumonia, bacterial pneumonia, viral pneumonia, atypical pneumonia, graft-versus-host disease (GVHD), COVID-19, reduction of the viral load of Covid-19, SARS-CoV1, sepsis, Ebola, bird flu, smallpox, or common flu.

34. A pharmaceutical composition according to claim 33, wherein the infection caused by a pathogen in the respiratory system is selected from viral pneumonia, bacterial pneumonia, or atypical pneumonia.

35. A pharmaceutical composition according to claim 34, wherein the infection caused by a pathogen in the respiratory system is a viral pneumonia.

36. A nutritional supplement or a medical food comprising at least one compound according to any one of claims 1 to 29, and the compounds 17-HDHA, and 18-HEPE, for use in improving the health of a patient suffering from an infection caused by a pathogen in the respiratory system.

37. A nutritional supplement or a medical food comprising at least one compound according to any one of claims 1 to 29, and the compounds 17-HDHA, and 18-HEPE, for use in reducing the viral load of a patient.

38. A nutritional supplement or a medical food according to any one of claims 36 to 37, wherein the compounds are in the free form, ethyl esters, triglycerides, diglycerides, monoglycerides, sphingolipids, phospholipids or as mixtures thereof.

39. A nutritional supplement or a food-medicine according to any one of claims 36 to 38, wherein the pathogen is Covid-19.

40. A method of diagnosing and/or monitoring the status of a patient suffering from a disease caused by the presence of a pathogen in the respiratory system based on the identification and quantification of the concentration of pro-resolution lipid mediators in blood, plasma, and/or serum from said patient, which are relevant in said disease.

41. A method for diagnosing and/or monitoring the status of a patient according to claim 40, wherein the relevant lipid mediators are RvE1, RvD2, RvD4, MaR1, and PDX.

42. A method of diagnosing and/or monitoring of the status of a patient according to claim 40, where it is determined whether a subject suffers from any of those pathologies described in the present invention when a decrease of at least 30% is observed, with respect to a healthy individual, of the concentration in blood, plasma and/or serum of each of the following pro-resolution mediators RvE1, RvD2, RvD4, MaR1, and PDX.

43. A method of diagnosing and/or monitoring of the status of a patient according to claim 40, where the relevant pro-resolution lipid mediators are RvE1, RvD2, RvD4, MaR1 and PDX, and the pro-inflammatory lipid mediators are PGD2 and LTB4.

44. A method of diagnosing and/or monitoring of the status of a patient according to claim 40, wherein the increase in the pro-inflammatory mediator LTB4 is at least 100%, with respect to a healthy individual.

45. A method for diagnosing and/or monitoring the status of a patient according to claim 40, wherein the measurement of the concentration values of the pro-resolution mediators is carried out in plasma.

46. A method of diagnosing and/or monitoring of the status of a patient according to claim 40, wherein the measurement of the concentration values of the pro-resolution mediators is carried out in serum.

47. A method for diagnosing and/or monitoring the status of a patient according to claim 46, wherein the blood sample is treated with an adenosine inhibitor selected from: adenosine deaminase (ADA), caffeine, 8-(3-chlorostyril)caffeine, 2-phenylaminoadenosine, 2-p-2-carboxyethylphenylamino-5'-N-ethylcarboxamide-adenosine, 5-N-ethylcarboxamide-adenosine, 5'-N-cyclopropyladenosine, 5'N-methylcarboxamide-adenosine, PD-1259444, 1,3- dipropyl-phenylxanthine, or 4- {2- [7-amino-2-(2-furyl)[1,2,4]-triazolo[2,3-a]-[1,3,5]-triazin-5-ilamine]ethyl}phenol (ZM 241385).

48. A method of diagnosing and/or monitoring of the status of a patient according to claim 40, wherein the disease caused by the presence of a pathogen in the respiratory system is selected from: pneumonia, bacterial pneumonia, viral pneumonia, or atypical pneumonia.

49. A method for diagnosing and/or monitoring the status of a patient according to claim 48, wherein the disease caused by the presence of a pathogen in the respiratory system is a viral pneumonia.

50. A method of diagnosing and/or monitoring of the status of a patient according to claim 40, wherein the disease caused by the presence of a pathogen in the respiratory system is selected from COVID-19, SARS-CoV1, sepsis, Ebola , bird flu, smallpox, or common flu.
